# EUROPEAN PATENT APPLICATION

(11) **EP 4 850 765 A1**
(43) Date of publication of application: **09.12.2026**
(21) Application number: 25744730.0
(22) Date of filing: 24.01.2025
(51) Int. Cl.: A61P 35/04, C07K 16/46, A61K 39/395

(54) **PHARMACEUTICAL USE OF ANTI-PD-1-ANTI-VEGFA BISPECIFIC ANTIBODY**

(30) Priority: 25.01.2024 CN 202410108261
(71) Applicant: Akeso Biopharma Co., Ltd., Zhongshan, Guangdong 528437 (CN)
(72) Inventor: XIA, Yu, Zhongshan, Guangdong 528437 (CN); YANG, Jie, Zhongshan, Guangdong 528437 (CN); LI, Wenting, Zhongshan, Guangdong 528437 (CN); XIA, Mengying, Zhongshan, Guangdong 528437 (CN); XIA, Kebin, Zhongshan, Guangdong 528437 (CN); LI, Baiyong, Zhongshan, Guangdong 528437 (CN)
(74) Representative: Gill Jennings & Every LLP
(86) International application number: PCT/CN2025/074723
(87) International publication number: WO 2025/157267

(57) **Abstract**

The present invention relates to the field of tumor therapy and molecular immunology, relates to a pharmaceutical use of an anti-PD-1-anti-VEGFA bispecific antibody, and in particular, to a use of the anti-PD-1-anti-VEGFA bispecific antibody or a pharmaceutical combination of said antibody and a chemotherapy drug in the preparation of a drug for treating non-small cell lung cancer with brain metastases. The bispecific antibody can effectively treat non-small cell lung cancer with brain metastases, has high safety, and has good prospects of application.

## Description

### CROSS-REFERENCE TO RELATED APPLICATIONS

The present application is based on Chinese Patent Application No. 202410108261.9 filed on January 25, 2024 and claims priority thereto, which is incorporated herein by reference in its entirety.

### TECHNICAL FIELD

The present disclosure pertains to the fields of tumor treatment and immunobiology, and relates to pharmaceutical use of an anti-PD-1/anti-VEGFA bispecific antibody. Specifically, the present disclosure relates to use of an anti-human PD-1/anti-human VEGFA bispecific antibody or a pharmaceutical combination thereof with chemotherapeutic drugs in preparing a medicament for treating brain metastases from non-small cell lung cancer.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor therapeutic effect, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

Vascular endothelial growth factor (VEGF) is a growth factor that can promote division and proliferation of endothelial cells, promote formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine manner, promote proliferation and migration of endothelial cells, induce angiogenesis, promote continuous growth of tumor, improve vascular permeability, cause fibrin deposition in surrounding tissues, and promote infiltration of mononuclear cells, fibroblasts, and endothelial cells, which facilitates the formation of tumor stroma and entry of tumor cells into new blood vessels, and promotes tumor metastases. Therefore, inhibiting tumor angiogenesis is considered as one of the most promising tumor treatment methods at present. The VEGF family includes VEGFA, VEGFB, VEGFC, VEGFD, and PIGF. Vascular endothelial growth factor receptors (VEGFRs) include VEGFR1 (also known as Flt1), VEGFR2 (also known as KDR or Flk1), VEGFR3 (also known as Flt4), and Neuropilin-1 (NRP-1). The first three receptors are members of the tyrosine kinase superfamily, and are of similar structures composed of an extramembrane region, a transmembrane segment, and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors. VEGFA mainly acts in combination with VEGFR1, VEGFR2, and NRP-1. VEGFR1 is the first identified receptor, and has higher affinity for VEGFA than VEGFR2 under normal physiological conditions but lower tyrosinase activity in the intracellular segment than VEGFR2 (Ma Li, Chinese Journal of Birth Health and Heredity, 24(5) (2016):146-148).

VEGFR2 is the primary regulator of angiogenesis and vascular development, and has much higher tyrosine kinase activity than VEGFR1. VEGFR2, after binding to ligand VEGFA, mediates the proliferation, differentiation, and other behaviors of vascular endothelial cells, as well as the formation process of blood vessels and the permeability of blood vessels (Roskoski R Jr. et al., Crit Rev Oncol Hematol, 62(3) (2007):179-213). VEGFA, after binding to VEGFR2, mediates the transcription and expression of related intracellular protein genes through the downstream PLC-γ-PKC-Raf-MEK-MAPK signaling pathway, and thus promotes the proliferation of vascular endothelial cells (Takahashi T et al., Oncogene, 18(13) (1999):2221-2230).

VEGFR3 is one of the tyrosine kinase family members, and is mainly expressed in embryonic vascular endothelial cells and mature lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein, and is incapable of independently transducing biological signals but able to mediate signaling only after forming a complex with a VEGF tyrosine kinase receptor. (Ma Li, Chinese Journal of Birth Health and Heredity, 24(5) (2016):146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase, infiltration to surrounding tissues, and other patterns of endothelial cells (Dong Hongchao et al., Sep. 2014, Journal of Modern Oncology, 22(9): 2231-3).

Currently, there are several humanized monoclonal antibodies targeting human VEGF, particularly VEGFA, such as bevacizumab, which has been approved by the U.S. Food and Drug Administration for the treatment of various tumors such as non-small cell lung cancer, renal cell carcinoma, cervical cancer, and metastatic colorectal cancer in succession during 2004.

The programmed cell death receptor-1 (PD-1), also known as CD279, is a type I transmembrane glycoprotein membrane surface receptor and a member of the CD28 immunoglobulin superfamily, and is commonly expressed in T cells, B cells, and myeloid cells. PD-1 has two natural ligands, PD-L1 and PD-L2. Both PD-L1 and PD-L2 are members of the B7 superfamily and are constitutively or inducibly expressed on the membrane surface of a variety of cells, including non-hematopoietic cells and a variety of tumor cells. PD-L1 is mainly expressed on T cells, B cells, DC and microvascular endothelial cells and a variety of tumor cells, while PD-L2 is expressed only on antigen-presenting cells such as dendritic cells and macrophages. The interaction between PD-1 and its ligands can inhibit the activation of lymph, the proliferation of T cells, and the secretion of cytokines such as IL-2 and IFN-γ.

A large number of studies have demonstrated that the tumor microenvironment can protect tumor cells from being damaged by immune cells, expression of PD-1 in lymphocytes infiltrating the tumor microenvironment is up-regulated, and various primary tumor tissues are PD-L1 positive in immunohistochemical analysis, such as lung cancer, liver cancer, ovarian cancer, skin cancer, colon cancer, and glioma. Meanwhile, the expression of PD-L1 in the tumor is significantly correlated with poor prognosis in cancer patients. Blocking the interaction between PD-1 and its ligands can promote the tumor-specific T cell immunity and enhance the immune elimination efficiency of tumor cells. A large number of clinical trials have shown that antibodies targeting PD-1 or PD-L1 can promote infiltration of CD8⁺ T cells into tumor tissues and up-regulate anti-tumor immune effector factors such as IL-2, IFN-γ, granzyme B, and perforin, thereby effectively inhibiting the growth of tumors.

In addition, anti-PD-1 antibodies may also be used in the treatment of viral chronic infections. Viral chronic infections are often accompanied by a loss of function of virus-specific effector T cells and a reduction in their number. The interaction between PD-1 and PD-L1 can be blocked by injecting a PD-1 antibody, thereby effectively inhibiting the exhaustion of effector T cells in viral chronic infection.

Due to the broad anti-tumor prospects and surprising efficacy of PD-1 antibodies, it is widely accepted in the industry that antibodies targeting the PD-1 pathway will bring breakthroughs in the treatment of a variety of tumors: for the treatment of non-small cell lung cancer, renal cell carcinoma, ovarian cancer, melanoma (Homet M. B., Parisi G., et al., Anti-PD-1 Therapy in Melanoma. Semin Oncol. Jun;42(3) (2015):466-473), lymphoma and anemia (Held SA, Heine A, et al., Advances in immunotherapy of chronic myeloid leukemia CML. Curr Cancer Drug Targets. Sep;13(7) (2013):768-74), or tumors with microsatellite instability-high (MSI-H) or deficient mismatch repair (dMMR) (several anti-PD-1 antibody drugs have been approved by the FDA or other regulatory authorities for the treatment of tumors with MSI-H/dMMR characteristics).

Bifunctional antibodies, also known as bispecific antibodies, are specific therapies that target two different antigens simultaneously, and can be produced by immunoselection purification. In addition, bispecific antibodies can also be produced by genetic engineering, which has certain advantages due to corresponding flexibility in aspects such as the optimization of binding sites, considerations of the synthetic form and yield. Currently, the bispecific antibody has been demonstrated to exist in over 45 forms (Müller D, Kontermann RE. Bispecific antibodies for cancer immunotherapy: current perspectives. BioDrugs, 2010; 24:89-98). A variety of bispecific antibodies developed at present are in the form of IgG-scFv, namely the Morrison form (Coloma M. J., Morrison S. L. Design and production of novel tetravalent bispecific antibodies. Nat Biotechnol., 1997;15:159-163). Due to its similarity to the naturally occurring IgG form, as well as its advantages in antibody engineering, expression, and purification, the IgG-scFv form has been proven to be an ideal form of bifunctional antibodies (Miller B. R., Demarest S. J., et al., Stability engineering of scFvs for the development of bispecific and multivalent antibodies. Protein Eng Des Sel 2010; 23:549-57; Fitzgerald J, Lugovskoy A. Rational engineering of antibody therapeutics targeting multiple oncogene pathways. MAbs 2011; 3:299-309).

Brain metastases (BM) in patients with malignant tumors often portend a poorer prognosis. Brain metastases may involve a variety of intracranial tissues, including brain parenchyma, spinal meninges, nerves, capillaries, and the like, and may also be accompanied by epilepsy, cognitive decline, sensory or motor dysfunction, and cranial neuropathy, which seriously reduces the quality of life of patients. The brain has a unique blood brain barrier (BBB) consisting of tightly connected non-fenestrated endothelial cells. Pericytes and the foot processes of astrocytes surround these non-fenestrated endothelial cells, providing support and maintaining the integrity of the blood brain barrier (Desland FA, Hormigo A. The CNS and the brain tumor microenvironment: implications for glioblastoma immunotherapy[J]. Int J Mol Sci, 2020, 21(19): 7358). In one aspect, the expression of endothelial cell adhesion molecules of the blood brain barrier is low, preventing peripheral immune cells from entering the central nervous system excessively; in another aspect, conventional chemotherapeutic drugs and targeted drugs also have difficulty crossing the blood brain barrier to enter the cranium for action (Daneman R, Zhou L, Agalliu D, et al. The mouse blood brain barrier transcriptome: a new resource for understanding the development and function of brain endothelial cells[J]. PLoS One, 2010, 5(10): e13741). However, the blood brain barrier is often damaged to a certain extent at the lesion site, enabling blood-derived tumor cells to invade intracranially, resulting in the occurrence of brain metastatic cancer.

A variety of peripheral immune cells including lymphocytes and neutrophils and various immune factors are involved in the development process of brain metastatic cancer, and these together constitute the immune microenvironment of the metastasis. Microglia activation can up-regulate interleukin (IL) expression upon the brain metastases, while IL-6 can exert immunosuppressive effects on effector T cells (Lin YJ, Wei KC, Chen PY, et al. Roles of neutrophils in glioma and brain metastases[J]. Front Immunol, 2021, 12:701383). The interaction between tumor cells and astrocytes can highly express IL-1β, thereby further activating the Notch signaling pathway. Activation of this pathway can increase the stemness of tumor stem cells and drive their proliferation and differentiation in the tumor microenvironment (Wang XC, Haaland B, Hu-Lieskovan S, et al. First line immunotherapy extends brain metastasis free survival, improves overall survival, and reduces the incidence of brain metastasis in patients with advanced melanoma[J]. CancerRep (Hoboken), 2021, 4(6): e1419).

Lung cancer is one of the common malignant tumors worldwide, and non-small cell lung cancer (NSCLC) is the most common subtype of lung cancer. 10%-15% of NSCLC patients have brain metastases at the first diagnosis, which causes various neurological symptoms, and 24%-44% of advanced NSCLC patients have brain metastases. The median survival time of treated patients after the first diagnosis of brain metastases from NSCLC is only 4-6 months. The vast majority of NSCLC patients with brain metastases are no longer suitable for surgical treatment at the time of diagnosis. Chemotherapy such as an antifolate formulation in combination with a platinum-based drug is still the main treatment for advanced NSCLC at present. However, due to the hindrance of the blood brain barrier, most chemotherapeutic drugs and targeted TKI-based drugs have limited efficacy on brain metastases. Third-generation TKI-based drugs have relatively strong anti-tumor activity and high blood brain barrier passing rates, and often have relatively good efficacy. However, such drugs are only suitable for some patients with insignificant symptoms of common classical driver gene mutations, and their application is also limited by drug resistance and safety.

At present, brain metastases from NSCLC are also treated clinically by immunotherapy. Studies have shown that immune checkpoint inhibitors (ICIs) can promote the recruitment of T cells (especially CD8⁺ T cells) from extracranial sites to the intracranial compartment, allowing more T cells to enter the brain to kill tumors (Taggart D, Andreou T, Scott KJ, Williams J, Rippaus N, Brownlie RJ, Ilett EJ, Salmond RJ, Melcher A, Lorger M. Anti-PD-1/anti-CTLA-4 efficacy in melanoma brain metastases depends on extracranial disease and augmentation of CD8+ T cell trafficking. Proc Natl Acad Sci U S A. 2018 Feb 13;115(7):E1540-E1549.). A study of durvalumab showed that in 25 EGFR-positive/ALK-positive brain metastasis patients, the objective response rate of driver gene-negative patients was 16.4%, which is higher than that of positive patients (12.2%). It can be seen that the efficacy of ICI monotherapy is not ideal for the overall patients with EGFR mutations (Garassino MC, Cho BC, Kim JH, et al. Durvalumab as third-line or later treatment for advanced non-small-cell lung cancer (ATLANTIC): an open-label, single-arm, phase2 study[J]. Lancet Oncol, 2018, 19(4): 521-536). In addition, there are immune checkpoint inhibitors used in combination with chemotherapy or multiple immune checkpoint inhibitors used in combination to treat brain metastases from lung cancer, but the incidence of immune-related adverse events (irAEs) is increased (Yang Shanru, Tai Risheng, Wang Geng, Qu Shutao, Lei Lei, Li Na. Problems associated with immune checkpoint inhibitor therapy-immune related adverse reactions irAEs[J]. Chinese Journal of Immunology, 2022, 38(16):2026-2030).

In summary, the development of new drugs or therapeutic approaches to treat brain metastases is of great clinical significance.

### SUMMARY

After intensive studies and creative efforts, the inventors have found that the anti-PD-1/anti-VEGFA bispecific antibody related to the present disclosure can effectively treat or prevent brain metastases from non-small cell lung cancer with or without chemotherapy, and has good safety.

The present disclosure is detailed below:
One aspect of the present disclosure relates to use of a bispecific antibody in preparing a medicament for treating or preventing brain metastases from non-small cell lung cancer, wherein
the bispecific antibody comprises:
   a first protein functional region targeting VEGFA, and
   a second protein functional region targeting PD-1,
   wherein
   the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
      or,
   the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

In the present disclosure, unless otherwise specified, the bispecific antibody is an anti-VEGFA/anti-PD-1 bispecific antibody, particularly an anti-human VEGFA/anti-human PD-1 bispecific antibody.

In some embodiments of the present disclosure, provided is the use, wherein the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

In some embodiments of the present disclosure, provided is the use, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

In some embodiments of the present disclosure, provided is the use, wherein the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

In some embodiments of the present disclosure, provided is the use, wherein the bispecific antibody is selected from any one of the following (7)-(12):
(7)
   an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
   an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
   an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
   an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
   an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
   an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

In some embodiments of the present disclosure, provided is the use, wherein the immunoglobulin is of a human IgG1 subtype.

In some embodiments of the present disclosure, provided is the use, wherein the immunoglobulin is of a human IgG1 subtype,
wherein, according to the EU numbering system, a heavy chain constant region of the immunoglobulin comprises one of the following mutation combinations:
L234A and L235A;
L234A and G237A;
L235A and G237A; or
L234A, L235A, and G237A.

In the present disclosure, letters before the position number represent amino acids before mutation, and letters after the position number represent amino acids after mutation, unless otherwise specified.

In some embodiments of the present disclosure, provided is the use, wherein according to the EU numbering system, the heavy chain constant region of the immunoglobulin further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In some embodiments of the present disclosure, provided is the use, wherein an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

In one or more embodiments of the present disclosure, the bispecific antibody is in the form of IgG-scFv, i.e., the Morrison format.

In one or more embodiments of the present disclosure, provided is the use, wherein
the heavy chain constant region of the immunoglobulin is a human Ig gamma-1 chain C region or a human Ig gamma-4 chain C region, and the light chain constant region of the immunoglobulin is a human Ig kappa chain C region.

In some embodiments of the present disclosure, the constant region of the immunoglobulin is humanized; for example, the heavy chain constant region is an Ig gamma-1 chain C region (ACCESSION: P01857); the light chain constant region is an Ig kappa chain C region (ACCESSION: P01834); or
the heavy chain constant region of the immunoglobulin is an Ig gamma-4 chain C region (ACCESSION: P01861.1); the light chain constant region is an Ig kappa chain C region (ACCESSION: P01834).

In some embodiments of the present disclosure, an amino acid sequence of the heavy chain constant region Ig gamma-1 chain C region (ACCESSION: P01857) is set forth in SEQ ID NO: 40.

In some embodiments of the present disclosure, an amino acid sequence of the heavy chain constant region Ig gamma-4 chain C region (ACCESSION: P01861.1) is set forth in SEQ ID NO: 41.

In some embodiments of the present disclosure, an amino acid sequence of the light chain constant region Ig kappa chain C region (ACCESSION: P01834) is set forth in SEQ ID NO: 42.

In some embodiments of the present disclosure, provided is the use, wherein the single-chain variable fragment is linked to the C-terminus of the heavy chain of the immunoglobulin. Since an immunoglobulin has two heavy chains, two single-chain variable fragment molecules are linked to one immunoglobulin molecule. Preferably, the two single-chain variable fragment molecules are identical.

In some embodiments of the present disclosure, provided is the use, wherein the single-chain variable fragment is two in number, and one terminus of each single-chain variable fragment is linked to the C-terminus or the N-terminus of one of the two heavy chains of the immunoglobulin.

In some embodiments of the present disclosure, a disulfide bond is present between the V_{H} and the V_{L} of the single-chain variable fragment. Methods for introducing a disulfide bond between the V_{H} and V_{L} of an antibody are well known in the art, see, e.g., U.S. Patent No. US5,747,654; Rajagopal et. al, Prot. Engin. 10(1997)1453-1459; Reiter et. al, Nat. Biotechnol. 14(1996)1239-1245; Reiter et. al, Protein Engineering 8(1995)1323-1331; Webber et. al, Molecular Immunology 32(1995)249-258; Reiter et. al, Immunity 2(1995)281-287; Reiter et. al, JBC 269(1994)18327-18331; Reiter et. al, Inter. J. of Cancer 58(1994)142-149; or Reiter et. al, Cancer Res.54(1994)2714-2718, which are incorporated herein by reference.

In some embodiments of the present disclosure, provided is the use, wherein the first protein functional region and the second protein functional region are linked directly or via a linker; and/or the heavy chain variable region of the single-chain variable fragment and the light chain variable region of the single-chain variable fragment are linked directly or via a linker.

In some embodiments of the present disclosure, provided is the use, wherein the linker is the polypeptide set forth in SEQ ID NO: 43 (GGGGS) or a polypeptide formed by concatenating a plurality of (e.g., 2, 3, 4, 5, or 6) polypeptides set forth in SEQ ID NO: 43.

In some embodiments of the present disclosure, provided is the use, wherein the first protein functional region and the second protein functional region are independently 1, 2, or more in number.

In some embodiments of the present disclosure, provided is the use, wherein the single-chain variable fragment is two in number, and the two single-chain variable fragments are linked to the C-termini of the two heavy chains of the immunoglobulin, respectively.

In some embodiments of the present disclosure, provided is the use, wherein the single-chain variable fragment is independently a sequential linkage of:
heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

In some embodiments of the present disclosure, provided is the use, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy
chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

In some embodiments of the present disclosure, provided is the use, wherein the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the use, wherein the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the use, wherein the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the use, wherein the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the use, wherein the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the use, wherein the bispecific antibody is the sole active ingredient.

In some embodiments of the present disclosure, provided is the use, which is use of a combination of the bispecific antibody with at least one chemotherapeutic drug in preparing a medicament for treating or preventing brain metastases from non-small cell lung cancer.

In some embodiments of the present disclosure, provided is the use, wherein the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

In some embodiments of the present disclosure, provided is the use, wherein a unit dose of the bispecific antibody is 100 mg-4000 mg, 200 mg-3000 mg, 200 mg-2000 mg, 300 mg-2000 mg, 400 mg-2000 mg, 500 mg-2000 mg, 500 mg-1000 mg, 500 mg-1500 mg, 600 mg-2000 mg, 800 mg-2000 mg, 1000 mg-2000 mg, or 1500 mg-2000 mg.

In some embodiments of the present disclosure, provided is the use, wherein a unit dose of the bispecific antibody is 100 mg-1000 mg, 200 mg-800 mg, 200 mg-500 mg, 300 mg-600 mg, 400 mg-500 mg, or 450 mg.

In some embodiments of the present disclosure, provided is the use, wherein a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight.

In some embodiments of the present disclosure, provided is the use, wherein the bispecific antibody is administered once every 1 week, every 2 weeks, every 3 weeks, or every 4 weeks.

In some embodiments of the present disclosure, provided is the use, wherein the bispecific antibody is administered by intravenous drip infusion, intravenous injection, or intraperitoneal injection.

In some embodiments of the present disclosure, provided is the use, wherein the combination is:
the bispecific antibody according to any one of the embodiments of the present disclosure, pemetrexed, and carboplatin; or
the bispecific antibody according to any one of the embodiments of the present disclosure, paclitaxel, and carboplatin.

In some embodiments of the present disclosure, provided is the use, wherein the combination is:
bispecific antibody VP101(hG1DM), pemetrexed, and carboplatin; or
bispecific antibody VP101(hG1DM), paclitaxel, and carboplatin.

In some embodiments of the present disclosure, provided is the use, wherein the combination is:
bispecific antibody VP101(hG1WT), pemetrexed, and carboplatin; or
bispecific antibody VP101(hG1WT), paclitaxel, and carboplatin.

In some embodiments of the present disclosure, provided is the use, wherein the combination is:
bispecific antibody VP101(hG4WT), pemetrexed, and carboplatin; or
bispecific antibody VP101(hG4WT), paclitaxel, and carboplatin.

In some embodiments of the present disclosure, provided is the use, which is characterized by one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

In some embodiments of the present disclosure, provided is the use, wherein the brain metastases from non-small cell lung cancer are selected from one or more of brain metastases from squamous non-small cell lung cancer, brain metastases from non-squamous non-small cell lung cancer, and brain metastases from lung adenocarcinoma.

In some embodiments of the present disclosure, provided is the use, wherein a patient with brain metastases from non-small cell lung cancer has PD-L1 TPS ≥ 50%, 1% < PD-L1 TPS ≥ 49%, or PD-L1 TPS ≤ 1%.

TPS is an abbreviation for a tumor cell proportion score.

In one or more embodiments of the present disclosure, the bispecific antibody is a humanized antibody.

In one or more embodiments of the present disclosure, the bispecific antibody is VP101(hG1WT), VP101(hG4WT), or VP101(hG1DM).

The medicament may be formulated into any dosage form known in the pharmaceutical field, such as tablets, pills, suspensions, emulsions, solutions, gels, capsules, powders, granules, elixirs, troches, suppositories, injections (including injection solutions, sterile powders for injection, and concentrated solutions for injection), inhalants, and sprays. The preferred dosage form depends on the intended route of administration and therapeutic use. The pharmaceutical composition of the present disclosure should be sterile and stable under the conditions of manufacture and storage. One preferred dosage form is an injection. Such injections may be sterile solutions for injection. For example, sterile solutions for injection can be prepared by the following method: a necessary dose of the bispecific antibody of the present disclosure is added in an appropriate solvent, and optionally, another desired ingredient (including, but not limited to, a pH regulator, a surfactant, an adjuvant, an ionic strength enhancer, an isotonic agent, a preservative, a diluent, or any combination thereof) is added at the same time, followed by filtration and sterilization. In addition, sterile solutions for injection can be prepared as sterile lyophilized powders (e.g., by vacuum drying or lyophilizing) for ease of storage and use. Such sterile lyophilized powders may be dispersed in a suitable carrier (e.g., sterile pyrogen-free water) prior to use.

In addition, the bispecific antibody of the present disclosure may be present in a medicament in a unit dose form for ease of administration. In certain embodiments, the unit dose is at least 1 mg, at least 5 mg, at least 10 mg, at least 15 mg, at least 20 mg, at least 25 mg, at least 30 mg, at least 45 mg, at least 50 mg, at least 75 mg, or at least 100 mg. When the pharmaceutical composition is in a liquid (e.g., injection) dosage form, it may comprise the bispecific antibody of the present disclosure at a concentration of at least 0.1 mg/mL, such as at least 0.25 mg/mL, at least 0.5 mg/mL, at least 1 mg/mL, at least 2.5 mg/mL, at least 5 mg/mL, at least 8 mg/mL, at least 10 mg/mL, at least 15 mg/mL, at least 25 mg/mL, at least 50 mg/mL, at least 75 mg/mL, or at least 100 mg/mL.

The bispecific antibody or the medicament of the present disclosure may be administered by any suitable method known in the art, including, but not limited to, oral, buccal, sublingual, ocular, parenteral, rectal, intrathecal, intracisternal, inguinal, intravesical, topical (e.g., a powder, an ointment, or a drop), or nasal route. However, for many therapeutic uses, the preferred route/mode of administration is parenteral (e.g., intravenous injection, subcutaneous injection, intraperitoneal injection, or intramuscular injection). Those skilled in the art will appreciate that the route and/or mode of administration will vary depending on the intended purpose. In one preferred embodiment, the bispecific antibody or the pharmaceutical composition of the present disclosure is administered by intravenous infusion or injection.

The bispecific antibody or the medicament provided in the present disclosure can be used alone or in combination, or used in combination with an additional pharmaceutically active agent (e.g., an anti-tumor chemotherapeutic drug). Such an additional pharmaceutically active agent may be administered prior to, concurrently with, or subsequent to administration of the bispecific antibody of the present disclosure or the pharmaceutical composition of the present disclosure.

In the present disclosure, the dosing regimen may be adjusted to achieve the optimal desired response (e.g., a therapeutic or prophylactic response). For example, the regimen may be a single dose, or multiple doses over a period of time, or the dose may be reduced or increased proportionally according to the emergency of the treatment.

In one or more embodiments of the present disclosure, one treatment cycle is 1 week, 2 weeks, 3 weeks, or 4 weeks. In some embodiments, one treatment cycle is 3 weeks.

Yet another aspect of the present disclosure relates to a bispecific antibody for use in treating or preventing brain metastases from non-small cell lung cancer, wherein
the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
   or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

In some embodiments of the present disclosure, provided is the bispecific antibody, which is for use in treating or preventing brain metastases from non-small cell lung cancer, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

In some embodiments of the present disclosure, provided is the bispecific antibody, which is for use in treating or preventing brain metastases from non-small cell lung cancer, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

In some embodiments of the present disclosure, provided is the bispecific antibody, which is for use in treating or preventing brain metastases from non-small cell lung cancer, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

In some embodiments of the present disclosure, provided is the bispecific antibody, which is for use in treating or preventing brain metastases from non-small cell lung cancer, wherein the bispecific antibody is selected from any one of the following (7)-(12):
(7)
   an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
   an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
   an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
   an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
   an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
   an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

In some embodiments of the present disclosure, provided is the bispecific antibody, which is for use in treating or preventing brain metastases from non-small cell lung cancer, wherein
the immunoglobulin is of a human IgG1 subtype,
wherein, according to the EU numbering system, a heavy chain constant region of the immunoglobulin comprises one of the following mutation combinations:
   L234A and L235A; or
   L234A, L235A, and G237A.

In some embodiments of the present disclosure, provided is the bispecific antibody, which is for use in treating or preventing brain metastases from non-small cell lung cancer, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

In some embodiments of the present disclosure, provided is the bispecific antibody, which is for use in treating or preventing brain metastases from non-small cell lung cancer, wherein
the first protein functional region and the second protein functional region are linked directly or via a linker; and/or the heavy chain variable region of the single-chain variable fragment and the light chain variable region of the single-chain variable fragment are linked directly or via a linker.

In some embodiments of the present disclosure, provided is the bispecific antibody, which is for use in treating or preventing brain metastases from non-small cell lung cancer, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

In some embodiments of the present disclosure, provided is the bispecific antibody, which is for use in treating or preventing brain metastases from non-small cell lung cancer, wherein
the first protein functional region and the second protein functional region are independently 1, 2, or more in number.

In some embodiments of the present disclosure, provided is the bispecific antibody, which is for use in treating or preventing brain metastases from non-small cell lung cancer, wherein
the single-chain variable fragment is two in number, and the two single-chain variable fragments are linked to the C-termini of the two heavy chains of the immunoglobulin, respectively.

In some embodiments of the present disclosure, provided is the bispecific antibody, which is for use in treating or preventing brain metastases from non-small cell lung cancer, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

In some embodiments of the present disclosure, provided is the bispecific antibody, which is for use in treating or preventing brain metastases from non-small cell lung cancer, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

In some embodiments of the present disclosure, provided is the bispecific antibody, which is for use in treating or preventing brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the bispecific antibody, which is for use in treating or preventing brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the bispecific antibody, which is for use in treating or preventing brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the bispecific antibody, which is for use in treating or preventing brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the bispecific antibody, which is for use in treating or preventing brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the bispecific antibody, which is for use in treating or preventing brain metastases from non-small cell lung cancer, wherein the bispecific antibody is administered in combination with at least one chemotherapeutic drug.

In some embodiments of the present disclosure, provided is the bispecific antibody, which is for use in treating or preventing brain metastases from non-small cell lung cancer, wherein the bispecific antibody is administered in combination with at least one chemotherapeutic drug,
and the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

In some embodiments of the present disclosure, provided is the bispecific antibody, which is characterized by one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

In some embodiments of the present disclosure, provided is the bispecific antibody, which is for use in treating or preventing brain metastases from non-small cell lung cancer, wherein
the bispecific antibody is administered in combination with pemetrexed and carboplatin; or
the bispecific antibody is administered in combination with paclitaxel and carboplatin.

In some embodiments of the present disclosure, provided is the bispecific antibody, which is for use in treating or preventing brain metastases from non-small cell lung cancer, is administered in combination with pemetrexed and carboplatin or in combination with paclitaxel and carboplatin, and is characterized by one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

In some embodiments of the present disclosure, provided is the bispecific antibody, wherein the brain metastases from non-small cell lung cancer are selected from one or more of brain metastases from squamous non-small cell lung cancer, brain metastases from non-squamous non-small cell lung cancer, and brain metastases from lung adenocarcinoma;
preferably, a patient with brain metastases from non-small cell lung cancer has PD-L1 TPS ≥ 50%, 1% < PD-L1 TPS ≥ 49%, or PD-L1 TPS ≤ 1%.

Another aspect of the present disclosure relates to a method for treating or preventing brain metastases from non-small cell lung cancer, which comprises a step of administering to a subject in need an effective amount of a bispecific antibody, wherein the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
   or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

In some embodiments of the present disclosure, provided is the method for treating or preventing the brain metastases from non-small cell lung cancer, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

In some embodiments of the present disclosure, provided is the method for treating or preventing the brain metastases from non-small cell lung cancer, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

In some embodiments of the present disclosure, provided is the method for treating or preventing the brain metastases from non-small cell lung cancer, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

In some embodiments of the present disclosure, provided is the method for treating or preventing the brain metastases from non-small cell lung cancer, wherein the bispecific antibody is selected from any one of the following (7)-(12):
(7)
   an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
   an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
   an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
   an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
   an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
   an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

In some embodiments of the present disclosure, provided is the method for treating or preventing the brain metastases from non-small cell lung cancer, wherein
the immunoglobulin is of a human IgG1 subtype.

In some embodiments of the present disclosure, provided is the method for treating or preventing the brain metastases from non-small cell lung cancer, wherein
the immunoglobulin is of a human IgG1 subtype,
wherein, according to the EU numbering system, a heavy chain constant region of the immunoglobulin comprises one of the following mutation combinations:
   L234A and L235A;
   L234A and G237A;
   L235A and G237A; or
   L234A, L235A, and G237A.

In some embodiments of the present disclosure, provided is the method for treating or preventing the brain metastases from non-small cell lung cancer, wherein according to the EU numbering system, the heavy chain constant region of the immunoglobulin further has one or more mutations selected from: N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In some embodiments of the present disclosure, provided is the method for treating or preventing the brain metastases from non-small cell lung cancer, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

In some embodiments of the present disclosure, provided is the method for treating or preventing the brain metastases from non-small cell lung cancer, wherein
the first protein functional region and the second protein functional region are linked directly or via a linker; and/or the heavy chain variable region of the single-chain variable fragment and the light chain variable region of the single-chain variable fragment are linked directly or via a linker.

In some embodiments of the present disclosure, provided is the method for treating or preventing the brain metastases from non-small cell lung cancer, wherein
the linker is the polypeptide set forth in SEQ ID NO: 43 (GGGGS) or a polypeptide formed by concatenating a plurality of (e.g., 2, 3, 4, 5, or 6) polypeptides set forth in SEQ ID NO: 43.

In some embodiments of the present disclosure, provided is the method for treating or preventing the brain metastases from non-small cell lung cancer, wherein
the first protein functional region and the second protein functional region are independently 1, 2, or more in number.

In some embodiments of the present disclosure, provided is the method for treating or preventing the brain metastases from non-small cell lung cancer, wherein
the single-chain variable fragment is two in number, and the two single-chain variable fragments are linked to the C-termini of the two heavy chains of the immunoglobulin, respectively.

In some embodiments of the present disclosure, provided is the method for treating or preventing the brain metastases from non-small cell lung cancer, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

In some embodiments of the present disclosure, provided is the method for treating or preventing the brain metastases from non-small cell lung cancer, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

In some embodiments of the present disclosure, provided is the method for treating or preventing the brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the method for treating or preventing the brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the method for treating or preventing the brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the method for treating or preventing the brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the method for treating or preventing the brain metastases from non-small cell lung cancer, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the method for treating or preventing the brain metastases from non-small cell lung cancer, wherein the bispecific antibody is the sole active ingredient.

In some embodiments of the present disclosure, provided is the method for treating or preventing the brain metastases from non-small cell lung cancer, which further comprises a step of administering to a subject in need an effective amount of at least one chemotherapeutic drug.

In some embodiments of the present disclosure, provided is the method for treating or preventing the brain metastases from non-small cell lung cancer, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

In some embodiments of the present disclosure, provided is the method for treating or preventing the brain metastases from non-small cell lung cancer, wherein the chemotherapeutic drug is administered concurrently with, prior to, or subsequent to administration of the bispecific antibody.

A typical non-limiting range of a therapeutically or prophylactically effective amount of the bispecific antibody of the present disclosure is 0.02-50 mg/kg, such as 0.1-50 mg/kg, 0.1-25 mg/kg, or 1-10 mg/kg. It should be noted that the dose may vary depending on the type and severity of the symptom to be treated. Furthermore, those skilled in the art will appreciate that for any particular patient, the particular dosing regimen may be adjusted over time according to the needs of the patient and the professional judgment of the physician; the dose ranges provided herein are for the illustrative purpose only and do not limit the use or scope of the pharmaceutical composition of the present disclosure.

In the present disclosure, the subject may be a mammal, such as a human.

In one or more embodiments of the present disclosure, provided is the method for treating or preventing the brain metastases from non-small cell lung cancer, wherein
a single administration dose of the anti-PD-1/anti-VEGFA bispecific antibody is 0.1-100 mg, preferably 1-50 mg (e.g., 1 mg, 2 mg, 3 mg, 4 mg, 5 mg, 6 mg, 7 mg, 8 mg, 9 mg, 10 mg, 10 mg-50 mg, 10 mg-40 mg, 10 mg-30 mg, 10 mg-20 mg, 10 mg-15 mg, 15 mg-30 mg, 15 mg-20 mg, 12 mg, 15 mg, 20 mg, 25 mg, or 30 mg) per kg body weight; or a single administration dose of the anti-PD-1/anti-VEGFA bispecific antibody is 10-5000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, about 1000 mg, about 1200 mg, about 1500 mg, about 1800 mg, about 2000 mg, about 2500 mg, or about 3000 mg), preferably 100-2500 mg, 500-2500 mg, 500-2000 mg, 500-2000 mg, or 500 mg-1500 mg in each subject;
preferably, drug administration is performed once every 3 days, every 4 days, every 5 days, every 6 days, every 10 days, every 1 week, every 2 weeks, every 3 weeks, or every 4 weeks;
preferably, the route of administration is intravenous drip infusion, intravenous injection, or intraperitoneal injection.

In some embodiments, the administration of the anti-PD-1/anti-VEGFA bispecific antibody is performed in cycles of 2 weeks (14 days) or 3 weeks (21 days), and preferably, the anti-PD-1/anti-VEGFA bispecific antibody is administered intravenously on the first day (D1) of each cycle. For example, the anti-PD-1/anti-VEGFA bispecific antibody is administered once every two weeks (q2w) or three weeks (q3w).

In some embodiments of the present disclosure, provided is the method for treating or preventing the brain metastases from non-small cell lung cancer, wherein a unit dose of the bispecific antibody is 100 mg-4000 mg, 200 mg-3000 mg, 200 mg-2000 mg, 300 mg-2000 mg, 400 mg-2000 mg, 500 mg-2000 mg, 500 mg-1000 mg, 500 mg-1500 mg, 600 mg-2000 mg, 800 mg-2000 mg, 1000 mg-2000 mg, or 1500 mg-2000 mg.

In one or more embodiments of the present disclosure, a unit dose of the bispecific antibody is 100 mg-1000 mg, 200 mg-800 mg, 200 mg-500 mg, 300 mg-600 mg, 400 mg-500 mg, or 450 mg.

In one or more embodiments of the present disclosure, a unit dose of the anti-tumor chemotherapeutic drug is 0.1 mg-100 mg, 0.5 mg-50 mg, 0.5 mg-10 mg, 1 mg-10 mg, 2 mg-8 mg, or 1 mg-5 mg.

In one or more embodiments of the present disclosure, a unit dose of the anti-tumor chemotherapeutic drug is 1 mg-20 mg, 2 mg-15 mg, 4 mg-12 mg, or 8 mg-12 mg.

In one or more embodiments of the present disclosure, a single administration dose of the anti-tumor chemotherapeutic drug is 10-1500 mg/m², 100-1000 mg/m², 200-900 mg/m², 300-800 mg/m², 400-700 mg/m², or 500-600 mg/m² based on a body surface area (m²) of each subject.

In one or more embodiments of the present disclosure, a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks.

In one or more embodiments of the present disclosure, carboplatin is administered once every 1 week, every 2 weeks, every 3 weeks, or every 4 weeks. In some embodiments, carboplatin is administered at a dose of AUC 2-6 min*mg/mL or AUC 2.5-5 min*mg/mL each time; preferably, carboplatin is administered once every 1 week, every 2 weeks, every 3 weeks, or every 4 weeks.

In one or more embodiments of the present disclosure, paclitaxel is administered once every 1 week, every 2 weeks, every 3 weeks, or every 4 weeks. In some embodiments, paclitaxel is administered at a dose of 67.5-175 mg/m², 87.5-175 mg/m², or 131-175 mg/m² each time; preferably, paclitaxel is administered once every 1 week, every 2 weeks, every 3 weeks, or every 4 weeks.

In some embodiments, non-small cell lung cancer is squamous non-small cell lung cancer (also known as squamous carcinoma, squamous cell carcinoma, squamous non-small cell lung cancer, or squamous epithelial cell carcinoma). In some embodiments, non-small cell lung cancer is non-small cell lung cancer that is histologically predominantly squamous cell carcinoma. In some embodiments, non-small cell lung cancer is non-squamous non-small cell lung cancer. In some embodiments, non-small cell lung cancer is adenocarcinoma (also known as lung adenocarcinoma). Preferably, non-small cell lung cancer is locally advanced, recurrent, and/or metastatic non-small cell lung cancer.

In one or more embodiments of the present disclosure, a patient with non-small cell lung cancer has PD-L1 TPS ≥ 50%, 1% < PD-L1 TPS ≥ 49%, or PD-L1 TPS ≤ 1%.

Yet another aspect of the present disclosure relates to a method for preparing a medicament for treating or preventing brain metastases from non-small cell lung cancer, which uses a bispecific antibody, wherein
the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively;
and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
   or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

In some embodiments of the present disclosure, provided is the preparation method, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

In some embodiments of the present disclosure, provided is the preparation method, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

In some embodiments of the present disclosure, provided is the preparation method, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

In some embodiments of the present disclosure, provided is the preparation method, wherein the bispecific antibody is selected from any one of the following (7)-(12):
(7)
   an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
   an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
   an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
   an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
   an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
   an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

In some embodiments of the present disclosure, provided is the preparation method, wherein
the immunoglobulin is of a human IgG1 subtype,
wherein, according to the EU numbering system, a heavy chain constant region of the immunoglobulin comprises one of the following mutation combinations:
   L234A and L235A; or
   L234A, L235A, and G237A.

In some embodiments of the present disclosure, provided is the preparation method, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

In some embodiments of the present disclosure, provided is the preparation method, wherein
the first protein functional region and the second protein functional region are linked directly or via a linker; and/or the heavy chain variable region of the single-chain variable fragment and the light chain variable region of the single-chain variable fragment are linked directly or via a linker.

In some embodiments of the present disclosure, provided is the preparation method, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

In some embodiments of the present disclosure, provided is the preparation method, wherein
the first protein functional region and the second protein functional region are independently 1, 2, or more in number.

In some embodiments of the present disclosure, provided is the preparation method, wherein
the single-chain variable fragment is two in number, and the two single-chain variable fragments are linked to the C-termini of the two heavy chains of the immunoglobulin, respectively.

In some embodiments of the present disclosure, provided is the preparation method, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

In some embodiments of the present disclosure, provided is the preparation method, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

In some embodiments of the present disclosure, provided is the preparation method, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the preparation method, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the preparation method, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the preparation method, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the preparation method, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the preparation method, wherein the bispecific antibody is administered in combination with at least one chemotherapeutic drug.

In some embodiments of the present disclosure, provided is the preparation method, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

In some embodiments of the present disclosure, provided is the preparation method, which is characterized by one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

In some embodiments of the present disclosure, provided is the preparation method, wherein
the bispecific antibody is administered in combination with pemetrexed and carboplatin; or
the bispecific antibody is administered in combination with paclitaxel and carboplatin.

In some embodiments of the present disclosure, provided is the preparation method, which is characterized by one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

In some embodiments of the present disclosure, provided is the preparation method, wherein the brain metastases from non-small cell lung cancer are selected from one or more of brain metastases from squamous non-small cell lung cancer, brain metastases from non-squamous non-small cell lung cancer, and brain metastases from lung adenocarcinoma;
preferably, a patient with brain metastases from non-small cell lung cancer has PD-L1 TPS ≥ 50%, 1% < PD-L1 TPS ≥ 49%, or PD-L1 TPS ≤ 1%.

Yet another aspect of the present disclosure relates to a pharmaceutical composition for treating or preventing brain metastases from non-small cell lung cancer, which comprises a bispecific antibody, wherein the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively;
and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
   or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

In some embodiments of the present disclosure, provided is the pharmaceutical composition, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

In some embodiments of the present disclosure, provided is the pharmaceutical composition, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

In some embodiments of the present disclosure, provided is the pharmaceutical composition, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

In some embodiments of the present disclosure, provided is the pharmaceutical composition, wherein the bispecific antibody is selected from any one of the following (7)-(12):
(7)
   an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
   an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
   an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
   an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
   an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
   an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

In some embodiments of the present disclosure, provided is the pharmaceutical composition, wherein
the immunoglobulin is of a human IgG1 subtype,
wherein, according to the EU numbering system, a heavy chain constant region of the immunoglobulin comprises one of the following mutation combinations:
   L234A and L235A; or
   L234A, L235A, and G237A.

In some embodiments of the present disclosure, provided is the pharmaceutical composition, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

In some embodiments of the present disclosure, provided is the pharmaceutical composition, wherein
the first protein functional region and the second protein functional region are linked directly or via a linker; and/or the heavy chain variable region of the single-chain variable fragment and the light chain variable region of the single-chain variable fragment are linked directly or via a linker.

In some embodiments of the present disclosure, provided is the pharmaceutical composition, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

In some embodiments of the present disclosure, provided is the pharmaceutical composition, wherein
the first protein functional region and the second protein functional region are independently 1, 2, or more in number.

In some embodiments of the present disclosure, provided is the pharmaceutical composition, wherein
the single-chain variable fragment is two in number, and the two single-chain variable fragments are linked to the C-termini of the two heavy chains of the immunoglobulin, respectively.

In some embodiments of the present disclosure, provided is the pharmaceutical composition, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

In some embodiments of the present disclosure, provided is the pharmaceutical composition, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

In some embodiments of the present disclosure, provided is the pharmaceutical composition, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the pharmaceutical composition, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the pharmaceutical composition, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18.

In some embodiments of the present disclosure, provided is the pharmaceutical composition, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the pharmaceutical composition, wherein
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

In some embodiments of the present disclosure, provided is the pharmaceutical composition, wherein the bispecific antibody is the sole active ingredient.

In some embodiments of the present disclosure, provided is the pharmaceutical composition, wherein the bispecific antibody is administered in combination with at least one chemotherapeutic drug.

In some embodiments of the present disclosure, provided is the pharmaceutical composition, wherein active ingredients are the bispecific antibody and at least one chemotherapeutic drug.

In some embodiments of the present disclosure, provided is the pharmaceutical composition, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

In some embodiments of the present disclosure, provided is the pharmaceutical composition, which further comprises one or more pharmaceutically acceptable auxiliary materials.

In some embodiments of the present disclosure, provided is the pharmaceutical composition, which consists of an active ingredient and one or more pharmaceutically acceptable auxiliary materials.

In some embodiments of the present disclosure, provided is the pharmaceutical composition, which consists of the bispecific antibody according to any one of the embodiments of the present disclosure and one or more pharmaceutically acceptable auxiliary materials.

In some embodiments of the present disclosure, provided is the pharmaceutical composition, which consists of the bispecific antibody according to any one of the embodiments of the present disclosure, one or more chemotherapeutic drugs, and one or more pharmaceutically acceptable auxiliary materials.

In some embodiments of the present disclosure, provided is the pharmaceutical composition, which is characterized by one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

In some embodiments of the present disclosure, provided is the pharmaceutical composition, which comprises:
co-administration of the bispecific antibody, pemetrexed, and carboplatin; or
co-administration of the bispecific antibody, paclitaxel, and carboplatin.

In some embodiments of the present disclosure, provided is the pharmaceutical composition, which is characterized by one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

In some embodiments of the present disclosure, provided is the pharmaceutical composition, wherein the brain metastases from non-small cell lung cancer are selected from one or more of brain metastases from squamous non-small cell lung cancer, brain metastases from non-squamous non-small cell lung cancer, and brain metastases from lung adenocarcinoma; preferably, a patient with brain metastases from non-small cell lung cancer has PD-L1 TPS ≥ 50%, 1% < PD-L1 TPS ≥ 49%, or PD-L1 TPS ≤ 1%.

Antibody drugs, especially monoclonal antibodies, have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light and heavy chains determine antigen binding; the variable region of each chain contains three hypervariable regions called complementarity determining regions (CDRs). The CDRs of the heavy chain (H Chain) comprise HCDR1, HCDR2, and HCDR3, and the CDRs of the light chain (L Chain) comprise LCDR1, LCDR2, and LCDR3, which are named by Kabat et al., see Bethesda M.d., Sequences of Proteins of Immunological Interest, Fifth Edition, NIH Publication (1-3) 1991: 91-3242.

Preferably, CDRs may also be defined by the IMGT numbering system, see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 38.suppl_1 (2009): D301-D307.

The amino acid sequences of the CDR regions of the monoclonal antibody sequences in (1)-(3) below were analyzed by technical means well known to those skilled in the art, for example, by the VBASE2 database and especially according to the IMGT definition, and the results are as follows:

### (1) Bevacizumab

An amino acid sequence of the heavy chain variable region is set forth in SEQ ID NO: 1, and an amino acid sequence of the light chain variable region is set forth in SEQ ID NO: 3.

Amino acid sequences of the 3 CDR regions of the heavy chain variable region are as follows:
HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)

Amino acid sequences of the 3 CDR regions of the light chain variable region are as follows:
LCDR1: QDISNY (SEQ ID NO: 31)
LCDR2: FTS (SEQ ID NO: 32)
LCDR3: QQYSTVPWT (SEQ ID NO: 33)

### (2) 14C12, 14C12H1L1, or 14C12H1L1(M)

Amino acid sequences of the 3 CDR regions of the heavy chain variable region are as follows:
HCDR1: GFAFSSYD (SEQ ID NO: 34)
HCDR2: ISGGGRYT (SEQ ID NO: 35)
HCDR3: ANRYGEAWFAY (SEQ ID NO: 36)

Amino acid sequences of the 3 CDR regions of the light chain variable region are as follows:
LCDR1: QDINTY (SEQ ID NO: 37)
LCDR2: RAN (SEQ ID NO: 38)
LCDR3: LQYDEFPLT (SEQ ID NO: 39)

### (3) VP101(hG1WT) or VP101(hG1DM)

Amino acid sequences of the 9 CDR regions of the heavy chain are as follows:
HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)
HCDR4: GFAFSSYD (SEQ ID NO: 34)
HCDR5: ISGGGRYT (SEQ ID NO: 35)
HCDR6: ANRYGEAWFAY (SEQ ID NO: 36)
HCDR7: QDINTY (SEQ ID NO: 37)
HCDR8: RAN (SEQ ID NO: 38)
HCDR9: LQYDEFPLT (SEQ ID NO: 39)

Amino acid sequences of the 3 CDR regions of the light chain variable region are as follows:
LCDR1: QDISNY (SEQ ID NO: 31)
LCDR2: FTS (SEQ ID NO: 32)
LCDR3: QQYSTVPWT (SEQ ID NO: 33).

For the antibody VP101(hG1DM) of the present disclosure, amino acid mutations are introduced into the non-variable region of VP101(hG1WT). According to the EU numbering system, amino acid mutations are introduced at positions 234 and 235:
VP101(hG1DM) was obtained by introducing a leucine-to-alanine point mutation at position 234 (L234A) and a leucine-to-alanine point mutation at position 235 (L235A) in the hinge region of the heavy chain.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, when referring to the amino acid sequence of the VEGFA protein (GenBank ID: NP_001165097.1), it includes the full length of the VEGFA protein, as well as a fusion protein of VEGFA, such as a fragment fused to an Fc protein fragment of a mouse or human IgG (mFc or hFc). However, those skilled in the art will appreciate that in the amino acid sequence of the VEGFA protein, mutations or variations (including but not limited to, substitutions, deletions, and/or additions) may naturally occur or can be artificially introduced without affecting biological functions thereof. Therefore, in the present disclosure, the term "VEGFA protein" shall include all such sequences, including their natural or artificial variants. In addition, when describing a sequence fragment of the VEGFA protein, it also includes the corresponding sequence fragments in their natural or artificial variants.

As used herein, when referring to the amino acid sequence of the VEGFR2 protein (also known as KDR, GenBank ID: NP_002244), it includes the full length of the VEGFR2 protein, or the extracellular fragment VEGFR2-ECD of VEGFR2, or a fragment comprising VEGFR2-ECD, and it also includes a fusion protein of VEGFR2-ECD, such as a fragment fused to an Fc protein fragment of a mouse or human IgG (mFc or hFc). However, those skilled in the art will appreciate that in the amino acid sequence of the VEGFR2 protein, mutations or variations (including but not limited to, substitutions, deletions, and/or additions) may naturally occur or can be artificially introduced without affecting biological functions thereof. Therefore, in the present disclosure, the term "VEGFR2 protein" shall include all such sequences, including their natural or artificial variants. In addition, when describing a sequence fragment of the VEGFR2 protein, it also includes the corresponding sequence fragments in their natural or artificial variants.

As used herein, unless otherwise specified, the VEGFR is VEGFR1 and/or VEGFR2; specific protein sequence thereof is a sequence known in the prior art, and reference may be made to the sequence disclosed in the existing literature or GenBank, such as VEGFR1 (VEGFR1, NCBI Gene ID: 2321); VEGFR2 (VEGFR2, NCBI Gene ID: 3791).

As used herein, when referring to the amino acid sequence of the PD-1 protein (programmed cell death protein 1, NCBI GenBank: NM_005018), it includes the full length of the PD-1 protein, or the extracellular fragment PD-1ECD of PD-1 or a fragment comprising PD-1ECD, and it also includes a fusion protein of PD-1ECD, such as a fragment fused to an Fc protein fragment of a mouse or human IgG (mFc or hFc). However, it will be appreciated by those skilled in the art that in the amino acid sequence of the PD-1 protein, mutations or variations (including but not limited to substitutions, deletions, and/or additions) may naturally occur or can be artificially introduced without affecting biological functions thereof. Therefore, in the present disclosure, the term "PD-1 protein" shall include all such sequences, including their natural or artificial variants. In addition, when describing a sequence fragment of the PD-1 protein, it also includes the corresponding sequence fragments in their natural or artificial variants.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into µ, δ, γ, α, and ε, and the antibodies are defined as IgM, IgD, IgG, IgA, and IgE isotypes, respectively. In light chains and heavy chains, the variable region and constant region are linked via a "J" region of about 12 or more amino acids, and the heavy chain further comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (called complementarity determining regions, or CDRs), and conservative regions called framework regions (FRs) are distributed between the CDRs. Each V_{H} and V_{L} consists of 3 CDRs and 4 FRs arranged from the amino-terminus to the carboxyl-terminus in the following order: FR1, CDR1, FR2, CDR2, FR3, CDR3, FR4. The variable regions (V_{H} and V_{L}) of each heavy chain/light chain pair form an antibody binding site. The assignment of amino acids to the regions or domains may be defined by Kabat Sequences of Proteins of Immunological Interest (National Institutes of Health, Bethesda, Md. (1987 and 1991)), or Chothia & Lesk J. Mol. Biol. 196(1987):901-917; Chothia et al. Nature 342(1989): 878-883 or the IMGT numbering system (see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 38.suppl_1 (2009): D301-D307). In particular, the heavy chain may further comprise more than 3 CDRs, such as 6, 9, or 12. For example, in the bispecific antibody of the present disclosure, the heavy chain may be an scFv with the C-terminus of the heavy chain of the IgG antibody linked to another antibody, and in this case, the heavy chain comprises 9 CDRs. The term "antibody" is not limited by any specific method for producing the antibody. For example, the antibody includes, in particular, a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the term "antigen-binding fragment" of the antibody, also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989)). An antigen-binding fragment of an antibody can be produced by recombinant DNA technique or by enzymatic or chemical cleavage of the intact antibody. In some cases, the antigen-binding fragment includes Fab, Fab', F (ab')₂, Fd, Fv, dAb, and a complementarity determining region (CDR) fragment, a single-chain variable fragment (e.g., scFv), a chimeric antibody, a diabody, and a polypeptide that comprises at least a portion of an antibody sufficient to impart specific antigen binding ability to the polypeptide.

As used herein, the term "Fd fragment" refers to an antibody fragment consisting of V_{H} and C_{H1} domains; the term "Fv fragment" refers to an antibody fragment consisting of the V_{L} and V_{H} domains of a single arm of an antibody; the term "dAb fragment" refers to an antibody fragment consisting of a V_{H} domain (Ward et al., Nature 341 (1989):544-546); the term "Fab fragment" refers to an antibody fragment consisting of V_{L}, V_{H}, C_{L}, and C_{H1} domains; the term "F(ab')₂ fragment" refers to an antibody fragment comprising two Fab fragments linked via the disulfide bridge on a hinge region.

In some cases, the antigen-binding fragment of the antibody is a single-chain variable fragment (e.g., scFv) in which the V_{L} and V_{H} domains are paired to form a monovalent molecule via a linker that enables the production of a single polypeptide chain (see, e.g., Bird et al., Science, 242 (1988):423-426 and Huston et al., Proc. Natl. Acad. Sci. USA, 85 (1988):5879-5883). Such scFv molecules may have a general structure: NH₂-V_{L}-linker-V_{H}-COOH or NH₂-V_{H}-linker-V_{L}-COOH. A suitable linker in the prior art consists of a repeated GGGGS (SEQ ID NO: 43) amino acid sequence or a variant thereof. For example, a linker having the amino acid sequence (GGGGS)₄ (SEQ ID NO: 18) may be used, but variants thereof may also be used (Holliger et al., Proc. Natl. Acad. Sci. USA 90 (1993): 6444-6448). Other linkers that can be used in the present disclosure are described in Alfthan et al., Protein Eng. 8 (1995):725-731, Choi et al., Eur. J. Immunol. 31 (2001): 94-106, Hu et al., Cancer Res. 56 (1996):3055-3061, Kipriyanov et al., J. Mol. Biol. 293 (1999):41-56, and Roovers et al., Cancer Immunol. (2001).

In some cases, the antigen-binding fragment of the antibody is a diabody, that is, a bivalent antibody, in which the V_{H} and V_{L} domains are expressed on a single polypeptide chain. However, the linker used is too short to allow the pairing of the two domains on the same chain. Thus the domains are forced to pair with the complementary domains on the other chain, and two antigen-binding sites are generated (see, e.g., Holliger P. et al., Proc. Natl. Acad. Sci. USA 90 (1993):6444-6448 and Poljak R. J. et al., Structure 2 (1994):1121-1123).

Antigen-binding fragments (e.g., the above-mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies. As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibody molecules, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained by hybridoma technique first reported by Kohler et al. (Nature, 256:495, 1975), and can also be obtained by recombinant DNA technique (for example, see U.S. Patent No. 4,816,567).

As used herein, the term "chimeric antibody" refers to an antibody in which one portion of the light chain or/and heavy chain is derived from one antibody (which may be derived from a specific species or belong to a specific antibody class or subclass), and another portion of the light chain or/and heavy chain is derived from another antibody (which may be derived from the same or different species or belong to the same or different antibody class or subclass); nonetheless, it retains binding activity against a target antigen (U.S. Patent 4,816,567 Cabilly et al.; Morrison et al., Proc. Natl. Acad. Sci. USA, 81 (1984):6851-6855).

As used herein, the term "humanized antibody" refers to an antibody or an antibody fragment obtained when all or a part of the CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 321 (1986):522-525; Reichmann et al., Nature, (1988) 332:323 329; Presta, Curr. Op. Struct. Biol., 2 (1992):593-596; and Clark, Immunol. Today 21 (2000): 397-402.

As used herein, the term "epitope" refers to a site on the antigen that an immunoglobulin or antibody specifically binds to. The "epitope" is also referred to in the art as an "antigenic determinant". The epitope or antigenic determinant generally consists of chemically active surface groups of molecules such as amino acids, carbohydrates, or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" or "being isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *Escherichia coli* or *Bacillus subtilis*, fungal cells such as yeast cells or *Aspergillus*, insect cells such as S2 Drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In certain embodiments, an antibody that specifically binds to a certain antigen (or an antibody that is specific for a certain antigen) refers to an antibody that binds the antigen with affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less. In some embodiments of the present disclosure, the term "target" refers to specific binding.

As used herein, the term "K_{D}" refers to a dissociation equilibrium constant for a specific antibody-antigen interaction and is used to describe the binding affinity between the antibody and the antigen. A smaller dissociation equilibrium constant indicates stronger antibody-antigen binding and higher affinity between the antibody and the antigen. Generally, an antibody binds to an antigen with a dissociation equilibrium constant (K_{D}) less than about 10⁻⁵ M, e.g., less than about 10⁻⁶ M, 10⁻⁷ M, 10⁻⁸ M, 10⁻⁹ M, or 10⁻¹⁰ M or less, for example, as measured by surface plasmon resonance (SPR) on a BIACORE instrument or by a Fortebio molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, in the present disclosure, amino acids are generally represented by single-letter and three-letter abbreviations known in the art. For example, alanine can be represented by A or Ala.

As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

In some embodiments, the anti-PD-1/anti-VEGFA bispecific antibody or the antigen-binding fragment thereof may be formulated together with one or more pharmaceutically acceptable carriers to prepare a suitable pharmaceutical composition.

As used herein, the term "effective amount" refers to an amount sufficient to obtain or at least partially obtain a desired effect. For example, a prophylactically effective amount (e.g., for a disease associated with PD-1 binding to PD-L1 or overexpression of VEGF, such as a tumor) is an amount sufficient to prevent, stop, or delay the onset of the disease (e.g., a disease associated with PD-1 binding to PD-L1 or overexpression of VEGF, such as a tumor); a therapeutically effective amount is an amount sufficient to cure or at least partially stop the disease and its complications in a patient suffering from the disease. It is well within the ability of those skilled in the art to determine such an effective amount. For example, the amount effective for the therapeutic purpose will depend on the severity of the disease to be treated, the overall state of the patient's own immune system, the general condition of the patient such as age, body weight, and gender, the route of administration, and other treatments given concurrently, etc.

### Beneficial Effects of the Present Disclosure

The present disclosure achieves any one or more of the following technical effects (1) to (4):
(1) The bispecific antibody of the present disclosure can effectively treat brain metastases from non-small cell lung cancer.
(2) The bispecific antibody of the present disclosure treats brain metastases from non-small cell lung cancer, and the median progression-free survival of patients is relatively long and can even reach 19.3 months.
(3) The bispecific antibody of the present disclosure exerts a rapid therapeutic effect on brain metastases from non-small cell lung cancer, and a partial response is achieved in some patients after 6 weeks of administration.
(4) The bispecific antibody of the present disclosure has good safety in treatment of cancer, particularly brain metastases from non-small cell lung cancer.

### BRIEF DESCRIPTION OF THE DRAWINGS

FIG. 1 shows cranial magnetic resonance imaging of a certain patient with the brain metastases from non-small cell lung cancer at 12 weeks after treatment with the anti-PD-1/anti-VEGFA bispecific antibody VP101(hG1DM) in combination with chemotherapeutic agents. The location marked with × inside the white circle in the baseline image represents the tumor lesion.
FIG. 2 shows cranial magnetic resonance imaging of a certain patient with the brain metastases from non-small cell lung cancer at 42 weeks after the anti-PD-1/anti-VEGFA bispecific antibody VP101(hG1DM) monotherapy.
FIG. 3 shows the intracranial response rate in patients with brain metastases from non-small cell lung cancer treated with the anti-PD-1/anti-VEGFA bispecific antibody VP101(hG1DM) (by RANO criteria).
FIG. 4 shows the median progression-free survival of patients with brain metastases from non-small cell lung cancer treated with the anti-PD-1/anti-VEGFA bispecific antibody VP101(hG1DM).

Some sequences involved in the present disclosure are shown in Table A below.

**Table A**

| SEQ ID NO: | Name | Sequence |
|---|---|---|
| 1 | Bevacizumab -VH | |
| 2 | Nucleic acid encoding bevacizumab-VH | GAGGTGCAGCTGGTCGAGTCCGGGGGGGGCT GGTGCAGCCAGGCGGGTCTCTGAGGCTGAGTT GCGCCGCTTCAGGGTACACCTTCACAAACTAT GGAATGAATTGGGTGCGCCAGGCACCAGGAAA GGGACTGGAGTGGGTCGGCTGGATCAACACTT ACACCGGGGAACCTACCTATGCAGCCGACTTT AAGCGGCGGTTCACCTTCAGCCTGGATACAAG CAAATCCACTGCCTACCTGCAGATGAACAGCC TGCGAGCTGAGGACACCGCAGTCTACTATTGT GCTAAATATCCCCACTACTATGGGAGCAGCCA TTGGTATTTTGACGTGTGGGGGCAGGGGACTC TGGTGACAGTGAGCAGC |
| 3 | Bevacizumab -VL | |
| 4 | Nucleic acid encoding bevacizumab-VL | GATATTCAGATGACTCAGAGCCCCTCCTCCCTG TCCGCCTCTGTGGGCGACAGGGTCACCATCAC ATGCAGTGCTTCACAGGATATTTCCAACTACCT GAATTGGTATCAGCAGAAGCCAGGAAAAGCAC CCAAGGTGCTGATCTACTTCACTAGCTCCCTGC ACTCAGGAGTGCCAAGCCGGTTCAGCGGATCC GGATCTGGAACCGACTTTACTCTGACCATTTCT AGTCTGCAGCCTGAGGATTTCGCTACATACTAT TGCCAGCAGTATTCTACCGTGCCATGGACATTT GGCCAGGGGACTAAAGTCGAGATCAAG |
| 5 | 14C12-VH | EVKLVESGGGLVKPGGSLKLSCAASGFAFSSYD MSWVRQTPEKRLEWVATISGGGRYTYYPDSVK GRFTISRDNARNTLYLQMSSLRSEDTALYYCANR YGEAWFAYWGQGTLVTVSA |
| 6 | Nucleic acid encoding 14C12-VH | GAGGTCAAACTGGTGGAGAGCGGCGGCGGGCT GGTGAAGCCCGGCGGGTCACTGAAACTGAGCT GCGCCGCTTCCGGCTTCGCCTTTAGCTCCTACG ACATGTCATGGGTGAGGCAGACCCCTGAGAAG CGCCTGGAATGGGTCGCTACTATCAGCGGAGG CGGGCGATACACCTACTATCCTGACTCTGTCAA AGGGAGATTCACAATTAGTCGGGATAACGCCA GAAATACTCTGTATCTGCAGATGTCTAGTCTGC GGTCCGAGGATACAGCTCTGTACTATTGTGCA AACCGGTACGGCGAAGCATGGTTTGCCTATTG GGGACAGGGCACCCTGGTGACAGTCTCTGCC |
| 7 | 14C12-VL | DIKMTQSPSSMYASLGERVTFTCKASQDINTYLS WFQQKPGKSPKTLIYRANRLVDGVPSRFSGSGSG QDYSLTISSLEYEDMGIYYCLQYDEFPLTFGAGT KLELK |
| 8 | Nucleic acid encoding 14C12-VL | GACATTAAGATGACACAGTCCCCTTCCTCAAT GTACGCTAGCCTGGGCGAGCGAGTGACCTTCA CATGCAAAGCATCCCAGGACATCAACACATAC CTGTCTTGGTTTCAGCAGAAGCCAGGCAAAAG CCCCAAGACCCTGATCTACCGGGCCAATAGAC TGGTGGACGGGGTCCCCAGCAGATTCTCCGGA TCTGGCAGTGGGCAGGATTACTCCCTGACCAT CAGCTCCCTGGAGTATGAAGACATGGGCATCT ACTATTGCCTGCAGTATGATGAGTTCCCTCTGA CCTTTGGAGCAGGCACAAAACTGGAACTGAAG |
| 9 | 14C12H1L1-VH | EVQLVESGGGLVQPGGSLRLSCAASGFAFSSYD MSWVRQAPGKGLDWVATISGGGRYTYYPDSVK GRFTISRDNSKNNLYLQMNSLRAEDTALYYCAN RYGEAWFAYWGQGTLVTVSS |
| 10 | Nucleic acid encoding 14C12H1L1-VH | GAAGTGCAGCTGGTCGAGTCTGGGGGAGGCT GGTGCAGCCCGGCGGGTCACTGCGACTGAGCT GCGCAGCTTCCGGATTCGCCTTTAGCTCCTACG ACATGTCCTGGGTGCGACAGGCACCAGGAAAG GGACTGGATTGGGTCGCTACTATCTCAGGAGG CGGGAGATACACCTACTATCCTGACAGCGTCA AGGGCCGGTTCACAATCTCTAGAGATAACAGT AAGAACAATCTGTATCTGCAGATGAACAGCCT GAGGGCTGAGGACACCGCACTGTACTATTGTG CCAACCGCTACGGGGAAGCATGGTTTGCCTAT TGGGGGCAGGGAACCCTGGTGACAGTCTCTAG T |
| 11 | 14C12H1L1-VL | DIQMTQSPSSMSASVGDRVTFTCRASQDINTYLS WFQQKPGKSPKTLIYRANRLVSGVPSRFSGSGSG QDYTLTISSLQPEDMATYYCLQYDEFPLTFGAGT KLELK |
| 12 | Nucleic acid encoding 14C12H1L1-VL | GACATTCAGATGACTCAGAGCCCCTCCTCCAT GTCCGCCTCTGTGGGCGACAGGGTCACCTTCA CATGCCGCGCTAGTCAGGATATCAACACCTAC CTGAGCTGGTTTCAGCAGAAGCCAGGGAAAAG CCCCAAGACACTGATCTACCGGGCTAATAGAC TGGTGTCTGGAGTCCCAAGTCGGTTCAGTGGCT CAGGGAGCGGACAGGACTACACTCTGACCATC AGCTCCCTGCAGCCTGAGGACATGGCAACCTA CTATTGCCTGCAGTATGATGAGTTCCCACTGAC CTTTGGCGCCGGGACAAAACTGGAGCTGAAG |
| 13 | 14C12H1L1-HC | EVQLVESGGGLVQPGGSLRLSCAASGFAFSSYD MSWVRQAPGKGLDWVATISGGGRYTYYPDSVK GRFTISRDNSKNNLYLQMNSLRAEDTALYYCAN RYGEAWFAYWGQGTLVTVSSASTKGPSVFPLAP SSKSTSGGTAALGCLVKDYFPEPVTVSWNSGALT SGVHTFPAVLQSSGLYSLSSVVTVPSSSLGTQTYI CNVNHKPSNTKVDKKVEPKSCDKTHTCPPCPAP ELLGGPSVFLFPPKPKDTLMISRTPEVTCVVVDVS HEDPEVKFNWYVDGVEVHNAKTKPREEQYNST YRVVSVLTVLHQDWLNGKEYKCKVSNKALPAPI EKTISKAKGQPREPQVYTLPPSRDELTKNQVSLT CLVKGFYPSDIAVEWESNGQPENNYKTTPPVLDS DGSFFLYSKLTVDKSRWQQGNVFSCSVMHEALH NHYTQKSLSLSPGK |
| 14 | Nucleic acid encoding 14C12H1L1-HC | GAAGTGCAGCTGGTCGAGTCTGGGGGAGGGCT GGTGCAGCCCGGCGGGTCACTGCGACTGAGCT GCGCAGCTTCCGGATTCGCCTTTAGCTCCTACG ACATGTCCTGGGTGCGACAGGCACCAGGAAAG GGACTGGATTGGGTCGCTACTATCTCAGGAGG CGGGAGATACACCTACTATCCTGACAGCGTCA AGGGCCGGTTCACAATCTCTAGAGATAACAGT AAGAACAATCTGTATCTGCAGATGAACAGCCT GAGGGCTGAGGACACCGCACTGTACTATTGTG CCAACCGCTACGGGGAAGCATGGTTTGCCTAT TGGGGGCAGGGAACCCTGGTGACAGTCTCTAG TGCCAGCACCAAAGGACCTAGCGTGTTTCCTCT CGCCCCCTCCTCCAAAAGCACCAGCGGAGGAA CCGCTGCTCTCGGATGTCTGGTGAAGGACTACT TCCCTGAACCCGTCACCGTGAGCTGGAATAGC GGCGCTCTGACAAGCGGAGTCCATACATTCCC TGCTGTGCTGCAAAGCAGCGGACTCTATTCCCT GTCCAGCGTCGTCACAGTGCCCAGCAGCAGCC TGGGCACCCAGACCTACATCTGTAACGTCAAC CACAAGCCCTCCAACACCAAGGTGGACAAGAA AGTGGAGCCCAAATCCTGCGACAAGACACACA CCTGTCCCCCCTGTCCTGCTCCCGAACTCCTCG GAGGCCCTAGCGTCTTCCTCTTTCCTCCCAAAC CCAAGGACACCCTCATGATCAGCAGAACCCCT GAAGTCACCTGTGTCGTCGTGGATGTCAGCCA TGAGGACCCCGAGGTGAAATTCAACTGGTATG TCGATGGCGTCGAGGTGCACAACGCCAAAACC AAGCCCAGGGAGGAACAGTACAACTCCACCTA CAGGGTGGTGTCCGTGCTGACAGTCCTCCACC AGGACTGGCTGAACGGCAAGGAGTACAAGTGC AAGGTGTCCAACAAGGCTCTCCCTGCCCCCATT GAGAAGACCATCAGCAAGGCCAAAGGCCAAC CCAGGGAGCCCCAGGTCTATACACTGCCTCCC TCCAGGGACGAACTCACCAAGAACCAGGTGTC CCTGACCTGCCTGGTCAAGGGCTTTTATCCCAG CGACATCGCCGTCGAGTGGGAGTCCAACGGAC AGCCCGAGAATAACTACAAGACCACCCCTCCT GTCCTCGACTCCGACGGCTCCTTCTTCCTGTAC AGCAAGCTGACCGTGGACAAAAGCAGGTGGC AGCAGGGAAACGTGTTCTCCTGCAGCGTGATG CACGAAGCCCTCCACAACCACTACACCCAGAA AAGCCTGTCCCTGAGCCCCGGCAAA |
| 15 | 14C12H1L1-LC | DIQMTQSPSSMSASVGDRVTFTCRASQDINTYLS WFQQKPGKSPKTLIYRANRLVSGVPSRFSGSGSG QDYTLTISSLQPEDMATYYCLQYDEFPLTFGAGT KLELKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSS PVTKSFNRGEC |
| 16 | Nucleic acid encoding 14C12H1L1-LC | GACATTCAGATGACTCAGAGCCCCTCCTCCAT GTCCGCCTCTGTGGGCGACAGGGTCACCTTCA CATGCCGCGCTAGTCAGGATATCAACACCTAC CTGAGCTGGTTTCAGCAGAAGCCAGGGAAAAG CCCCAAGACACTGATCTACCGGGCTAATAGAC TGGTGTCTGGAGTCCCAAGTCGGTTCAGTGGCT CAGGGAGCGGACAGGACTACACTCTGACCATC AGCTCCCTGCAGCCTGAGGACATGGCAACCTA CTATTGCCTGCAGTATGATGAGTTCCCACTGAC CTTTGGCGCCGGGACAAAACTGGAGCTGAAGC GAACTGTGGCCGCTCCCTCCGTCTTCATTTTC CCCCTTCTGACGAACAGCTGAAATCAGGCACA GCCAGCGTGGTCTGTCTGCTGAACAATTTCTAC CCTAGAGAGGCAAAAGTGCAGTGGAAGGTCG ATAACGCCCTGCAGTCCGGCAACAGCCAGGAG AGTGTGACTGAACAGGACTCAAAAGATAGCAC CTATTCCCTGTCTAGTACACTGACTCTGTCCAA GGCTGATTACGAGAAGCACAAAGTGTATGCAT GCGAAGTGACACATCAGGGACTGTCAAGCCCC GTGACTAAGTCTTTTAACCGGGGCGAATGT |
| 17 | 14C12H1L1( M)-VL | DIQMTQSPSSMSASVGDRVTFTCRASQDINTYLS WFQQKPGKSPKTLIYRANRLVSGVPSRFSGSGSG QDYTLTISSLQPEDMATYYCLQYDEFPLTFGAGT KLELKR |
| 18 | Linker1 | GGGGSGGGGSGGGGSGGGGS |
| 19 | Linker2 | GGGGSGGGGSGGGGS |
| 20 | Heavy chain of immunoglob ulin portion in VP101(hG1 WT) | EVQLVESGGGLVQPGGSLRLSCAASGYTFTNYG MNWVRQAPGKGLEWVGWINTYTGEPTYAADFK RRFTFSLDTSKSTAYLQMNSLRAEDTAVYYCAK YPHYYGSSHWYFDVWGQGTLVTVSSASTKGPSV FPLAPSSKSTSGGTAALGCLVKDYFPEPVTVSWN SGALTSGVHTFPAVLQSSGLYSLSSVVTVPSSSLG TQTYICNVNHKPSNTKVDKKVEPKSCDKTHTCPP CPAPELLGGPSVFLFPPKPKDTLMISRTPEVTCVV VDVSHEDPEVKFNWYVDGVEVHNAKTKPREEQ YNSTYRVVSVLTVLHQDWLNGKEYKCKVSNKA LPAPIEKTISKAKGQPREPQVYTLPPSRDELTKNQ VSLTCLVKGFYPSDIAVEWESNGQPENNYKTTPP VLDSDGSFFLYSKLTVDKSRWQQGNVFSCSVMH EALHNHYTQKSLSLSPGK |
| 21 | Nucleic acid encoding heavy chain of immunoglob ulin portion in VP101(hG1 WT) | GAGGTGCAGCTGGTCGAGTCCGGGGGGGGCT GGTGCAGCCAGGCGGGTCTCTGAGGCTGAGTT GCGCCGCTTCAGGGTACACCTTCACAAACTAT GGAATGAATTGGGTGCGCCAGGCACCAGGAAA GGGACTGGAGTGGGTCGGCTGGATCAACACTT ACACCGGGGAACCTACCTATGCAGCCGACTTT AAGCGGCGGTTCACCTTCAGCCTGGATACAAG CAAATCCACTGCCTACCTGCAGATGAACAGCC TGCGAGCTGAGGACACCGCAGTCTACTATTGT GCTAAATATCCCCACTACTATGGGAGCAGCCA TTGGTATTTTGACGTGTGGGGGCAGGGGACTC TGGTGACAGTGAGCAGCGCAAGCACCAAAGG GCCCAGCGTGTTTCCTCTCGCCCCCTCCTCCAA AAGCACCAGCGGAGGAACCGCTGCTCTCGGAT GTCTGGTGAAGGACTACTTCCCTGAACCCGTC ACCGTGAGCTGGAATAGCGGCGCTCTGACAAG CGGAGTCCATACATTCCCTGCTGTGCTGCAAA GCAGCGGACTCTATTCCCTGTCCAGCGTCGTCA CAGTGCCCAGCAGCAGCCTGGGCACCCAGACC TACATCTGTAACGTCAACCACAAGCCCTCCAA CACCAAGGTGGACAAGAAAGTGGAGCCCAAA TCCTGCGACAAGACACACACCTGTCCCCCCTGT CCTGCTCCCGAACTCCTCGGAGGCCCTAGCGTC TTCCTCTTTCCTCCCAAACCCAAGGACACCCTC ATGATCAGCAGAACCCCTGAAGTCACCTGTGT CGTCGTGGATGTCAGCCATGAGGACCCCGAGG TGAAATTCAACTGGTATGTCGATGGCGTCGAG GTGCACAACGCCAAAACCAAGCCCAGGGAGG AACAGTACAACTCCACCTACAGGGTGGTGTCC GTGCTGACAGTCCTCCACCAGGACTGGCTGAA CGGCAAGGAGTACAAGTGCAAGGTGTCCAACA AGGCTCTCCCTGCCCCCATTGAGAAGACCATC AGCAAGGCCAAAGGCCAACCCAGGGAGCCCC AGGTCTATACACTGCCTCCCTCCAGGGACGAA CTCACCAAGAACCAGGTGTCCCTGACCTGCCT GGTCAAGGGCTTTTATCCCAGCGACATCGCCG TCGAGTGGGAGTCCAACGGACAGCCCGAGAAT AACTACAAGACCACCCCTCCTGTCCTCGACTCC GACGGCTCCTTCTTCCTGTACAGCAAACTGACC GTCGATAAATCTAGGTGGCAGCAGGGCAACGT GTTCTCTTGTTCCGTGATGCATGAAGCACTGCA CAACCATTATACCCAGAAGTCTCTGAGCCTGTC CCCCGGCAAG |
| 22 | Heavy chain of immunoglob ulin portion in VP101(hG4 WT) | EVQLVESGGGLVQPGGSLRLSCAASGYTFTNYG MNWVRQAPGKGLEWVGWINTYTGEPTYAADFK RRFTFSLDTSKSTAYLQMNSLRAEDTAVYYCAK YPHYYGSSHWYFDVWGQGTLVTVSSASTKGPSV FPLAPCSRSTSESTAALGCLVKDYFPEPVTVSWN SGALTSGVHTFPAVLQSSGLYSLSSVVTVPSSSLG TKTYTCNVDHKPSNTKVDKRVESKYGPPCPPCP APEFLGGPSVFLFPPKPKDTLMISRTPEVTCVVVD VSQEDPEVQFNWYVDGVEVHNAKTKPREEQFNS TYRVVSVLTVLHQDWLNGKEYKCKVSNKGLPSS IEKTISKAKGQPREPQVYTLPPSQEEMTKNQVSLT CLVKGFYPSDIAVEWESNGQPENNYKTTPPVLDS DGSFFLYSRLTVDKSRWQEGNVFSCSVMHEALH NHYTQKSLSLSLGK |
| 23 | Nucleic acid encoding heavy chain of immunoglob ulin portion in VP101(hG4 WT) | GAGGTGCAGCTGGTCGAGTCCGGGGGGGGCT GGTGCAGCCAGGCGGGTCTCTGAGGCTGAGTT GCGCCGCTTCAGGGTACACCTTCACAAACTAT GGAATGAATTGGGTGCGCCAGGCACCAGGAAA GGGACTGGAGTGGGTCGGCTGGATCAACACTT ACACCGGGGAACCTACCTATGCAGCCGACTTT AAGCGGCGGTTCACCTTCAGCCTGGATACAAG CAAATCCACTGCCTACCTGCAGATGAACAGCC TGCGAGCTGAGGACACCGCAGTCTACTATTGT GCTAAATATCCCCACTACTATGGGAGCAGCCA TTGGTATTTTGACGTGTGGGGGCAGGGGACTC TGGTGACAGTGAGCAGCGCAAGCACCAAAGG GCCCTCGGTCTTCCCCCTGGCGCCCTGCTCCAG GAGCACCTCCGAGAGCACAGCCGCCCTGGGCT GCCTGGTCAAGGACTACTTCCCCGAACCGGTG ACGGTGTCGTGGAACTCAGGCGCCCTGACCAG CGGCGTGCACACCTTCCCGGCTGTCCTACAGTC CTCAGGACTCTACTCCCTCAGCAGCGTGGTGA CCGTGCCCTCCAGCAGCTTGGGCACGAAGACC TACACCTGCAACGTAGATCACAAGCCCAGCAA CACCAAGGTGGACAAGAGAGTTGAGTCCAAAT ATGGTCCCCCATGCCCACCATGCCCAGCACCT GAGTTCCTGGGGGGACCATCAGTCTTCCTGTTC CCCCCAAAACCCAAGGACACTCTCATGATCTC CCGGACCCCTGAGGTCACGTGCGTGGTGGTGG ACGTGAGCCAGGAAGACCCCGAGGTCCAGTTC AACTGGTACGTGGATGGCGTGGAGGTGCATAA TGCCAAGACAAAGCCGCGGGAGGAGCAGTTCA ACAGCACGTACCGTGTGGTCAGCGTCCTCACC GTCCTGCACCAGGACTGGCTGAACGGCAAGGA GTACAAGTGCAAGGTCTCCAACAAAGGCCTCC CGTCCTCCATCGAGAAAACCATCTCCAAAGCC AAAGGGCAGCCCCGAGAGCCACAGGTGTACAC CCTGCCCCCATCCCAGGAGGAGATGACCAAGA ACCAGGTCAGCCTGACCTGCCTGGTCAAAGGC TTCTACCCCAGCGACATCGCCGTGGAGTGGGA GAGCAATGGGCAGCCGGAGAACAACTACAAG ACCACGCCTCCCGTGCTGGACTCCGACGGCTC CTTCTTCCTCTACAGCAGGCTAACCGTGGACAA GAGCAGGTGGCAGGAGGGGAATGTCTTCTCAT GCTCCGTGATGCATGAGGCTCTGCACAACCAC TACACACAGAAGTCTCTGAGCCTGTCCCTCGG CAAG |
| 24 | Heavy chain of immunoglob ulin portion in VP101(hG1 DM) | EVQLVESGGGLVQPGGSLRLSCAASGYTFTNYG MNWVRQAPGKGLEWVGWINTYTGEPTYAADFK RRFTFSLDTSKSTAYLQMNSLRAEDTAVYYCAK YPHYYGSSHWYFDVWGQGTLVTVSSASTKGPSV FPLAPSSKSTSGGTAALGCLVKDYFPEPVTVSWN SGALTSGVHTFPAVLQSSGLYSLSSVVTVPSSSLG TQTYICNVNHKPSNTKVDKKVEPKSCDKTHTCPP CPAPEAAGGPSVFLFPPKPKDTLMISRTPEVTCVV VDVSHEDPEVKFNWYVDGVEVHNAKTKPREEQ YNSTYRVVSVLTVLHQDWLNGKEYKCKVSNKA LPAPIEKTISKAKGQPREPQVYTLPPSRDELTKNQ VSLTCLVKGFYPSDIAVEWESNGQPENNYKTTPP VLDSDGSFFLYSKLTVDKSRWQQGNVFSCSVMH EALHNHYTQKSLSLSPGK |
| 25 | Nucleic acid encoding heavy chain of immunoglobulin portion in VP101(hG1 DM) | GAGGTGCAGCTGGTCGAGTCCGGGGGGGGCT GGTGCAGCCAGGCGGGTCTCTGAGGCTGAGTT GCGCCGCTTCAGGGTACACCTTCACAAACTAT GGAATGAATTGGGTGCGCCAGGCACCAGGAAA GGGACTGGAGTGGGTCGGCTGGATCAACACTT ACACCGGGGAACCTACCTATGCAGCCGACTTT AAGCGGCGGTTCACCTTCAGCCTGGATACAAG CAAATCCACTGCCTACCTGCAGATGAACAGCC TGCGAGCTGAGGACACCGCAGTCTACTATTGT GCTAAATATCCCCACTACTATGGGAGCAGCCA TTGGTATTTTGACGTGTGGGGGCAGGGGACTC TGGTGACAGTGAGCAGCGCAAGCACCAAAGG GCCCAGCGTGTTTCCTCTCGCCCCCTCCTCCAA AAGCACCAGCGGAGGAACCGCTGCTCTCGGAT GTCTGGTGAAGGACTACTTCCCTGAACCCGTC ACCGTGAGCTGGAATAGCGGCGCTCTGACAAG CGGAGTCCATACATTCCCTGCTGTGCTGCAAA GCAGCGGACTCTATTCCCTGTCCAGCGTCGTCA CAGTGCCCAGCAGCAGCCTGGGCACCCAGACC TACATCTGTAACGTCAACCACAAGCCCTCCAA CACCAAGGTGGACAAGAAAGTGGAGCCCAAA TCCTGCGACAAGACACACACCTGTCCCCCCTGT CCTGCTCCCGAAGCTGCTGGAGGCCCTAGCGT CTTCCTCTTTCCTCCCAAACCCAAGGACACCCT CATGATCAGCAGAACCCCTGAAGTCACCTGTG TCGTCGTGGATGTCAGCCATGAGGACCCCGAG GTGAAATTCAACTGGTATGTCGATGGCGTCGA GGTGCACAACGCCAAAACCAAGCCCAGGGAG GAACAGTACAACTCCACCTACAGGGTGGTGTC CGTGCTGACAGTCCTCCACCAGGACTGGCTGA ACGGCAAGGAGTACAAGTGCAAGGTGTCCAAC AAGGCTCTCCCTGCCCCCATTGAGAAGACCAT CAGCAAGGCCAAAGGCCAACCCAGGGAGCCC CAGGTCTATACACTGCCTCCCTCCAGGGACGA ACTCACCAAGAACCAGGTGTCCCTGACCTGCC TGGTCAAGGGCTTTTATCCCAGCGACATCGCC GTCGAGTGGGAGTCCAACGGACAGCCCGAGAA TAACTACAAGACCACCCCTCCTGTCCTCGACTC CGACGGCTCCTTCTTCCTGTACAGCAAACTGAC CGTCGATAAATCTAGGTGGCAGCAGGGCAACG TGTTCTCTTGTTCCGTGATGCATGAAGCACTGC ACAACCATTATACCCAGAAGTCTCTGAGCCTG TCCCCCGGCAAG |
| 26 | Light chain of immunoglob ulin portion in VP101(hG1 DM) | DIQMTQSPSSLSASVGDRVTITCSASQDISNYLNW YQQKPGKAPKVLIYFTSSLHSGVPSRFSGSGSGT DFTLTISSLQPEDFATYYCQQYSTVPWTFGQGTK VEIKRTVAAPSVFIFPPSDEQLKSGTASVVCLLNN FYPREAKVQWKVDNALQSGNSQESVTEQDSKDS TYSLSSTLTLSKADYEKHKVYACEVTHQGLSSPV TKSFNRGEC |
| 27 | Nucleic acid encoding light chain of immunoglob ulin portion in VP101(hG1 DM) | GATATTCAGATGACTCAGAGCCCCTCCTCCCTG TCCGCCTCTGTGGGCGACAGGGTCACCATCAC ATGCAGTGCTTCACAGGATATTTCCAACTACCT GAATTGGTATCAGCAGAAGCCAGGAAAAGCAC CCAAGGTGCTGATCTACTTCACTAGCTCCCTGC ACTCAGGAGTGCCAAGCCGGTTCAGCGGATCC GGATCTGGAACCGACTTTACTCTGACCATTTCT AGTCTGCAGCCTGAGGATTTCGCTACATACTAT TGCCAGCAGTATTCTACCGTGCCATGGACATTT GGCCAGGGGACTAAAGTCGAGATCAAGCGGA CCGTGGCCGCTCCCAGTGTCTTCATTTTCCCC CTAGCGACGAACAGCTGAAATCCGGACAGCC TCTGTGGTCTGTCTGCTGAACAACTTCTACCCT AGAGAGGCAAAAGTGCAGTGGAAGGTCGATA ACGCCCTGCAGAGTGGCAATTCACAGGAGAGC GTGACAGAACAGGACTCCAAAGATTCTACTTA TAGTCTGTCAAGCACACTGACTCTGAGCAAGG CTGACTACGAAAAGCATAAAGTGTATGCATGT GAGGTCACCCACCAGGGGCTGAGCAGTCCAGT CACCAAGTCATTCAACAGAGGCGAGTGC |
| 28 | Bevacizumab -HCDR1 | GYTFTNYG |
| 29 | Bevacizumab -HCDR2 | INTYTGEP |
| 30 | Bevacizumab -HCDR3 | AKYPHYYGSSHWYFDV |
| 31 | Bevacizumab -LCDR1 | QDISNY |
| 32 | Bevacizumab -LCDR2 | FTS |
| 33 | Bevacizumab -LCDR3 | QQYSTVPWT |
| 34 | 14C12-HCDR1 | GFAFSSYD |
| 35 | 14C12-HCDR2 | ISGGGRYT |
| 36 | 14C12-HCDR3 | ANRYGEAWFAY |
| 37 | 14C12-LCDR1 | QDINTY |
| 38 | 14C12-LCDR2 | RAN |
| 39 | 14C12-LCDR3 | LQYDEFPLT |
| 40 | Ig gamma-1 chain C region | |
| 41 | Ig gamma-4 chain C region | ASTKGPSVFPLAPCSRSTSESTAALGCLVKDYFPE PVTVSWNSGALTSGVHTFPAVLQSSGLYSLSSVV TVPSSSLGTKTYTCNVDHKPSNTKVDKRVESKY GPPCPSCPAPEFLGGPSVFLFPPKPKDTLMISRTPE VTCVVVDVSQEDPEVQFNWYVDGVEVHNAKTK PREEQFNSTYRVVSVLTVLHQDWLNGKEYKCKV SNKGLPSSIEKTISKAKGQPREPQVYTLPPSQEEM TKNQVSLTCLVKGFYPSDIAVEWESNGQPENNY KTTPPVLDSDGSFFLYSRLTVDKSRWQEGNVFSC SVMHEALHNHYTQKSLSLSLGK |
| 42 | Ig kappa chain C region | |
| | | NRGEC |
| 43 | Linker | GGGGS |
| 44 | Heavy chain of immunoglobu lin portion + linker + scFv portion in VP101(hG1D M) (equivalent to heavy chain of bispecific antibody) | EVQLVESGGGLVQPGGSLRLSCAASGYTFTNYG MNWVRQAPGKGLEWVGWINTYTGEPTYAADFK RRFTFSLDTSKSTAYLQMNSLRAEDTAVYYCAK YPHYYGSSHWYFDVWGQGTLVTVSSASTKGPSV FPLAPSSKSTSGGTAALGCLVKDYFPEPVTVSWN SGALTSGVHTFPAVLQSSGLYSLSSVVTVPSSSLG TQTYICNVNHKPSNTKVDKKVEPKSCDKTHTCPP CPAPEAAGGPSVFLFPPKPKDTLMISRTPEVTCVV VDVSHEDPEVKFNWYVDGVEVHNAKTKPREEQ YNSTYRVVSVLTVLHQDWLNGKEYKCKVSNKA LPAPIEKTISKAKGQPREPQVYTLPPSRDELTKNQ VSLTCLVKGFYPSDIAVEWESNGQPENNYKTTPP VLDSDGSFFLYSKLTVDKSRWQQGNVFSCSVMH EALHNHYTQKSLSLSPGKGGGGSGGGGSGGGGS GGGGSEVQLVESGGGLVQPGGSLRLSCAASGFA FSSYDMSWVRQAPGKGLDWVATISGGGRYTYY PDSVKGRFTISRDNSKNNLYLQMNSLRAEDTAL YYCANRYGEAWFAYWGQGTLVTVSSGGGGSGG GGSGGGGSGGGGSDIQMTQSPSSMSASVGDRVT FTCRASQDINTYLSWFQQKPGKSPKTLIYRANRL VSGVPSRFSGSGSGQDYTLTISSLQPEDMATYYC LQYDEFPLTFGAGTKLELKR |
| 45 | scFv 1 (VH-linker-VL) | EVQLVESGGGLVQPGGSLRLSCAASGFAFSSYD MSWVRQAPGKGLDWVATISGGGRYTYYPDSVK GRFTISRDNSKNNLYLQMNSLRAEDTALYYCAN RYGEAWFAYWGQGTLVTVSSGGGGSGGGGSGG GGSGGGGSDIQMTQSPSSMSASVGDRVTFTCRAS QDINTYLSWFQQKPGKSPKTLIYRANRLVSGVPS RFSGSGSGQDYTLTISSLQPEDMATYYCLQYDEF PLTFGAGTKLELKR |
| 46 | scFv 2 (VL-linker-VH) | DIQMTQSPSSMSASVGDRVTFTCRASQDINTYLS WFQQKPGKSPKTLIYRANRLVSGVPSRFSGSGSG QDYTLTISSLQPEDMATYYCLQYDEFPLTFGAGT KLELKRGGGGSGGGGSGGGGSGGGGSEVQLVES GGGLVQPGGSLRLSCAASGFAFSSYDMSWVRQA PGKGLDWVATISGGGRYTYYPDSVKGRFTISRD NSKNNLYLQMNSLRAEDTALYYCANRYGEAWF AYWGQGTLVTVSS |
| 47 | scFv 3 (VH-linker-VL) | EVQLVESGGGLVQPGGSLRLSCAASGFAFSSYD MSWVRQAPGKGLDWVATISGGGRYTYYPDSVK GRFTISRDNSKNNLYLQMNSLRAEDTALYYCAN RYGEAWFAYWGQGTLVTVSSGGGGSGGGGSGG GGSDIQMTQSPSSMSASVGDRVTFTCRASQDINT YLSWFQQKPGKSPKTLIYRANRLVSGVPSRFSGS GSGQDYTLTISSLQPEDMATYYCLQYDEFPLTFG AGTKLELKR |
| 48 | scFv 4 (VL-linker-VH) | DIQMTQSPSSMSASVGDRVTFTCRASQDINTYLS WFQQKPGKSPKTLIYRANRLVSGVPSRFSGSGSG QDYTLTISSLQPEDMATYYCLQYDEFPLTFGAGT KLELKRGGGGSGGGGSGGGGSEVQLVESGGGLV QPGGSLRLSCAASGFAFSSYDMSWVRQAPGKGL DWVATISGGGRYTYYPDSVKGRFTISRDNSKNN LYLQMNSLRAEDTALYYCANRYGEAWFAYWG QGTLVTVSS |

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Guide to Molecular Cloning Experiments, authored by J. Sambrook et al., and translated by Huang Peitang et al., third edition, Science Press) or according to the package insert. Reagents or instruments used are all commercially available conventional products if the manufacturers thereof are not specified.

### Preparation Example 1: Preparation of Anti-VEGFA Antibody Bevacizumab

For the amino acid sequences of the heavy chain variable region and the light chain variable region of the commercially available anti-VEGFA monoclonal antibody Avastin (bevacizumab), refer to the Chinese Patent Publication No. CN1259962A. Genscript was entrusted to synthesize the nucleotide sequences encoding the heavy chain variable region and the light chain variable region. The amino acid sequence of the heavy chain variable region of bevacizumab (bevacizumab-VH) was set forth in SEQ ID NO: 1.

The nucleotide sequence encoding the heavy chain variable region of bevacizumab was set forth in SEQ ID NO: 2.

The amino acid sequence of the light chain variable region of bevacizumab (bevacizumab-VL) was set forth in SEQ ID NO: 3.

The nucleotide sequence encoding the light chain variable region of bevacizumab was set forth in SEQ ID NO: 4.

The heavy chain constant regions were all the Ig gamma-1 chain C region (ACCESSION: P01857); the light chain constant regions were all the Ig kappa chain C region (ACCESSION: P01834).

The heavy chain cDNA and the light chain cDNA of bevacizumab were cloned into vector pcDNA3.1, and the recombinant expression plasmid of the antibody bevacizumab was obtained. The recombinant plasmid was transfected into 293F cells. The 293F cell culture solution was purified and then tested.

The anti-VEGFA monoclonal antibody Avastin (bevacizumab) was thus obtained.

### Preparation Example 2: Sequence Design of Anti-PD-1 Antibody 14C12, Humanized Antibody 14C12H1L1 Thereof, and Mutant 14C12H1L1(M)

The amino acid sequences and encoding nucleotide sequences of the heavy and light chains of the anti-PD-1 antibody 14C12 and its humanized antibody 14C12H1L1 were identical to those of 14C12 and 14C12H1L1 in Chinese Patent Publication No. CN 106967172A, respectively.

### (1) Heavy and light chain variable region sequences of 14C12

The amino acid sequence of the heavy chain variable region of 14C12 was set forth in SEQ ID NO: 5.

The nucleotide sequence encoding the heavy chain variable region of 14C12 was set forth in SEQ ID NO: 6.

The amino acid sequence of the light chain variable region of 14C12 was set forth in SEQ ID NO: 7.

The nucleotide sequence encoding the light chain variable region of 14C12 was set forth in SEQ ID NO: 8.

### (2) Heavy and light chain variable region sequences and heavy and light chain sequences of humanized monoclonal antibody 14C12H1L1

The amino acid sequence of the heavy chain variable region of 14C12H1L1 was set forth in SEQ ID NO: 9.

The nucleotide sequence encoding the heavy chain variable region of 14C12H1L1 was set forth in SEQ ID NO: 10.

The amino acid sequence of the light chain variable region of 14C12H1L1 was set forth in SEQ ID NO: 11.

The nucleotide sequence encoding the light chain variable region of 14C12H1L1 was set forth in SEQ ID NO: 12.

The amino acid sequence of the heavy chain (14C12H1) of 14C12H1L1 was set forth in SEQ ID NO: 13.

The nucleotide sequence encoding the heavy chain (14C12H1) of 14C12H1L1 was set forth in SEQ ID NO: 14.

The amino acid sequence of the light chain (14C12L1) of 14C12H1L1 was set forth in SEQ ID NO: 15.

The nucleotide sequence encoding the light chain (14C12L1) of 14C12H1L1 was set forth in SEQ ID NO: 16.

### (3) Heavy and light chain variable region sequences of 14C12H1L1(M)

14C12H1L1(M) was obtained by mutating certain amino acids in the framework region (light chain) on the basis of 14C12H1L1.

The amino acid sequence of the light chain variable region 14C12L1(M) of 14C12H1L1(M) was set forth in SEQ ID NO: 17.

The amino acid sequence of the heavy chain variable region 14C12H1(M) of 14C12H1L1(M) was identical to the heavy chain variable region 14C12H1 of 14C12H1L1, as set forth in SEQ ID NO: 9.

### Preparation Example 3: Sequence Design of Bispecific Antibodies

### 1. Sequence design

The structure of the bispecific antibodies of the present disclosure was in the Morrison format (IgG-scFv), i.e., the C-termini of two heavy chains of an IgG antibody were each linked to an scFv fragment of another antibody, and the main composition design of the heavy and light chains is as shown in Table 1 below.

On the basis of bevacizumab described above, the VP101 antibody comprised the amino acid sequences of the heavy and light chain variable regions of 14C12H1L1(M) as the scFv fragments, and was referred to as VP101(M). Compared with 14C12H1L1, 14C12H1L1(M) demonstrated an effectively optimized bispecific antibody structure and improved efficacy.

**Table 1: Composition design of heavy and light chains of VP101(M) and VP101(G4M)**

| Bispecific antibody name | Immunoglobulin portion | | Linker fragment | scFv portion |
|---|---|---|---|---|
| | Heavy chain | Light chain | | |
| VP101(M) | Bevacizu mab-H | Bevacizu mab-L | Linker1 | 14C12H1(M)_{V}- Linker1-14C12L1(M)_{V} |
| VP101(G4M) | Bevacizu mab-G4H | Bevacizu mab-L | Linker1 | 14C12H1(M)_{V}- Linker1-14C12L1(M)_{V} |

In Table 1 above:
(1) Those with the "V" label at the lower right corner refer to the variable region of the corresponding heavy chain or the variable region of the corresponding light chain. For those without the "V" label, the corresponding heavy or light chain is the full length comprising the constant region. The amino acid sequences of these variable regions or full-length sequences and the nucleotide sequences encoding them were as described in the corresponding sequences in the preparation examples described above.
(2) The amino acid sequence of Linker1 was set forth in SEQ ID NO: 18. Optionally, the amino acid sequence of Linker2 was set forth in SEQ ID NO: 19. Linker2 may replace the Linker1 described above.
(3) Bevacizumab-H employed the Ig gamma-1 chain C region (ACCESSION: P01857) as the heavy chain constant region.
(4) Bevacizumab-G4H employed the Ig gamma-4 chain C region (ACCESSION: P01861.1) as the heavy chain constant region.

### 2. Expression and purification of antibody VP101(M)

The heavy chain cDNA sequence and the light chain cDNA sequence of VP101(M) were each cloned into vector pUC57simple (provided by Genscript) to obtain plasmids pUC57simple-VP101H and pUC57simple-VP101L, respectively.

Plasmids pUC57simple-VP101H and pUC57simple-VP101L were enzyme-digested (HindIII&EcoRI), and heavy and light chains isolated by electrophoresis were separately subcloned into vector pcDNA3.1, and recombinant plasmids were extracted to co-transfect 293F cells. After 7 days of cell culture, the culture solution was separated by high-speed centrifugation, and the supernatant was concentrated and loaded onto a HiTrap MabSelect SuRe column. The protein was eluted in one step with an elution buffer. The target sample, antibody VP101, was isolated and buffer-exchanged into PBS.

### 3. Test of antibody VP101(M)

The purified sample was separately added to a reduced protein electrophoresis loading buffer and a non-reduced protein electrophoresis loading buffer, boiled, and subjected to SDS-PAGE electrophoresis analysis.

For differentiation from the mutant antibody in Preparation Example 4, VP101(M) is also referred to as VP101(hG1WT) in the present disclosure. VP101(M) described above was the "wild-type", which employed the Ig gamma-1 chain C region (ACCESSION: P01857) as the heavy chain constant region and the Ig kappa chain C region (ACCESSION: P01834) as the light chain constant region.

The amino acid sequence of the heavy chain of the immunoglobulin portion in VP101(hG1WT) was set forth in SEQ ID NO: 20.

The nucleotide sequence encoding the heavy chain of the immunoglobulin portion in VP101(hG1WT) was set forth in SEQ ID NO: 21.

For differentiation from the mutant antibody in Preparation Example 4, VP101(G4M) is also referred to as VP101(hG4WT) in the present disclosure. VP101(G4M) described above was the "wild-type", which employed the Ig gamma-4 chain C region (ACCESSION: P01861.1) as the heavy chain constant region and the Ig kappa chain C region (ACCESSION: P01834) as the light chain constant region.

The amino acid sequence of the heavy chain of the immunoglobulin portion in VP101(hG4WT) was set forth in SEQ ID NO: 22.

The nucleotide sequence encoding the heavy chain of the immunoglobulin portion in VP101(hG4WT) was set forth in SEQ ID NO: 23.

### Preparation Example 4: Non-Variable Region Amino Acid Mutation Design Based on Humanized Bispecific Antibody VP101(hG1WT)

On the basis of VP101(hG1WT) obtained in Preparation Example 3, the inventors obtained VP101(hG1DM) by introducing a leucine-to-alanine point mutation at position 234 (L234A) and a leucine-to-alanine point mutation at position 235 (L235A) in the heavy chain.

The amino acid sequence of the heavy chain of the immunoglobulin portion in VP101(hG1DM) was set forth in SEQ ID NO: 24.

The nucleotide sequence encoding the heavy chain of the immunoglobulin portion in VP101(hG1DM) was set forth in SEQ ID NO: 25.

The amino acid sequences of the light chains of the immunoglobulin portions of VP101(hG1DM), VP101(hG1WT), and VP101(hG4WT) were identical, and their encoding nucleotide sequences were also identical.

The amino acid sequence of the light chain of the immunoglobulin portion in VP101(hG1DM) was set forth in SEQ ID NO: 26.

The nucleotide sequence encoding the light chain of the immunoglobulin portion in VP101(hG1DM) was set forth in SEQ ID NO: 27.

The amino acid sequence of the "heavy chain of immunoglobulin portion + linker + scFv portion" in the anti-PD-1/anti-VEGFA bispecific antibody VP101(hG1DM) (equivalent to the heavy chain of the bispecific antibody) was set forth in SEQ ID NO: 44.

The amino acid sequence of the scFv was set forth in SEQ ID NO: 45.

### Example 1: Effective Treatment of Anti-PD-1/Anti-VEGFA Bispecific Antibody VP101(hG1DM) in Patients with Brain Metastases from Non-Small Cell Lung Cancer

Patients with advanced NSCLC from trials AK112-201 (NCT04736823) and AK112-202 (NCT04900363) had not received systemic treatment for cancer, had a performance status score of 0-1, were aged between 18 and 75 years, and were eligible for treatment with VP101(hG1DM) in combination with chemotherapeutic agents or VP101(hG1DM) monotherapy, respectively. The research and development code of VP101(hG1DM) was AK112.

Each administration cycle was defined as three weeks. On the first day of each cycle, drugs were administered in the following order: intravenous injection of AK112 first, followed by intravenous injection of pemetrexed or paclitaxel, and finally intravenous injection of carboplatin.

The dosing regimen for the trial No. AK112-201 was as follows:
Patients with non-squamous non-small cell lung cancer were treated with VP101(hG1DM) (20 mg/kg, Q3W) in combination with pemetrexed (500 mg/m²) and carboplatin (AUC 5 min*mg/mL, Q3W) for a total of 4 cycles. If the disease did not progress, VP101(hG1DM) (20 mg/kg, Q3W) in combination with pemetrexed (500 mg/m²) was administered for maintenance therapy for 2 years.

Patients with squamous non-small cell lung cancer were treated with VP101(hG1DM) (20 mg/kg, Q3W) in combination with paclitaxel (175 mg/m²) and carboplatin (AUC 5 min*mg/mL, Q3W) for 4 cycles. If the disease did not progress, VP101(hG1DM) (20 mg/kg, Q3W) was administered for maintenance therapy for 2 years.

The dosing regimen for the trial No. AK112-202 was as follows:
Patients were treated with VP101(hG1DM) (20 mg/kg, Q3W) for a total of 4 cycles. If the disease did not progress, VP101(hG1DM) (20 mg/kg, Q3W) was administered for maintenance therapy for 2 years.

Initial examination for brain metastases included cranial MRI or head CT to confirm the presence of brain metastases. Patients with brain metastases at baseline were eligible if they had no symptoms caused by brain metastases or were clinically stable for at least 2 weeks after treatment. Patients with brain metastases who had received radiotherapy or were unable to receive subsequent magnetic resonance imaging due to allergy could not participate in the assessment of the intracranial activity of the anti-PD-1/anti-VEGFA bispecific antibody. For patients with brain metastases at baseline levels, brain magnetic resonance imaging was performed every 6 weeks during treatment. In addition to the response of extracranial target lesions assessed according to the RECIST 1.1 criteria, brain metastases were serially assessed by 2 independent neuroradiologists (from the United States and China) according to the Response Assessment in Neuro-Oncology (RANO) criteria.

A total of 35 patients met the criteria for inclusion in this analysis: 28 from the trial AK112-201 and 7 from the trial AK112-202. These patients had a median age of 60 years (42-69 years). Among the 35 patients, 77% were male; 26 patients (74.3%) had non-small cell lung adenocarcinoma, 5 patients (14.3%) had squamous non-small cell lung cancer, and 4 patients (11.4%) had other types of NSCLC. Additionally, 46% of the 35 patients were positive for PD-L1 expression (PD-L1 TPS ≥ 1%).

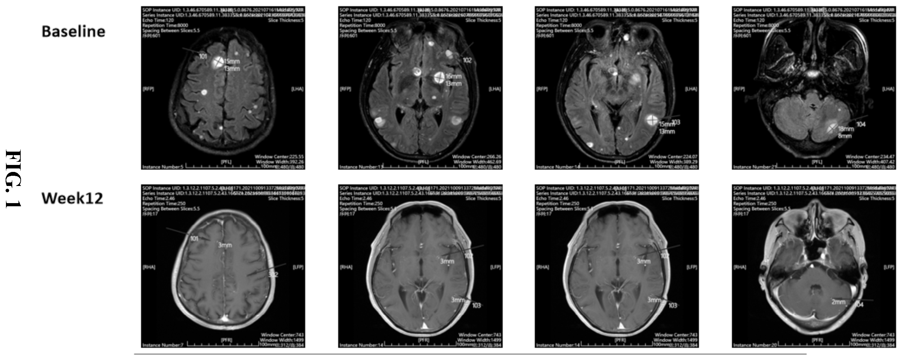

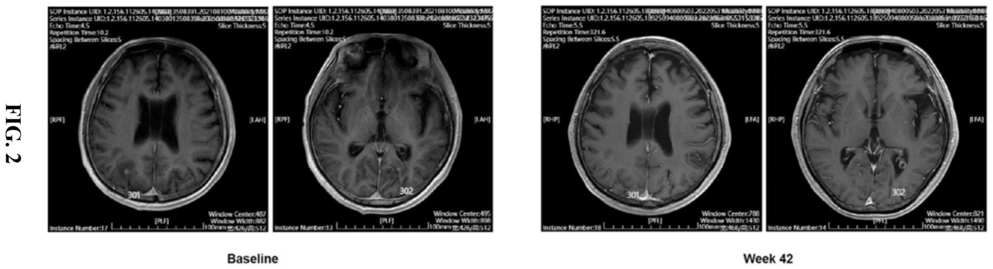

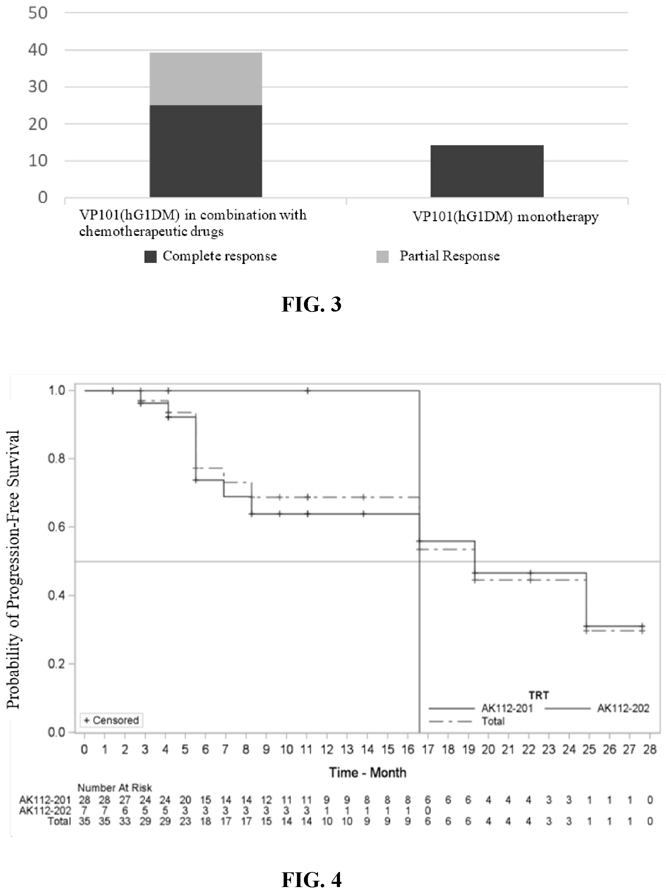

The results showed that a total of 12 patients (34%) with brain metastases from non-small cell lung cancer achieved responses of intracranial tumor lesions, among which 8 patients achieved complete responses of intracranial tumor lesions. Of the 12 patients with brain metastases from non-small cell lung cancer, 11 patients (39%) received treatment with VP101(hG1DM) in combination with chemotherapeutic drugs and 1 patient (14%) received VP101(hG1DM) monotherapy. Of the 8 patients who achieved complete responses of intracranial tumor lesions, 7 patients (25% of the total number of patients in the trial AK112-201) received treatment with VP101(hG1DM) in combination with chemotherapeutic drugs, and 1 patient (14% of the total number of patients in the trial AK112-202) received VP101(hG1DM) monotherapy, as shown in FIG. 3. The patients with brain metastases in the two cohorts had a median progression-free survival of 19.3 months (as shown in FIG. 4), and the patients with brain metastases did not have intracranial hemorrhage within 3 months after treatment. Some patients achieved partial responses at week 6 of the administration. As an example, the results of one patient with the brain metastases from non-small cell lung cancer receiving treatment with VP101(hG1DM) in combination with chemotherapeutic drugs are shown in Table 2 and FIG. 1. The results showed that tumor lesions of the brain metastases of the patient with non-small cell lung cancer were effectively alleviated. In addition, the results of another patient with the brain metastases from non-small cell lung cancer receiving VP101(hG1DM) monotherapy are shown in FIG. 2. The results showed that tumor lesions of the brain metastases of the patient with non-small cell lung cancer were also effectively alleviated.

**Table 2. Assessment results of intracranial tumor lesions in the same patient with brain metastases from non-small cell lung cancer as shown in FIG. 1**

| Tumor assessment | Week 6 | Week 12 | Week 18 |
|---|---|---|---|
| Overall (RANO-BM) | Partial Response | Partial Response | Partial Response |

### Example 2: In Vivo Safety Analysis of Anti-PD-1/Anti-VEGFA Bispecific Antibody

The safety and efficacy of the anti-PD-1/anti-VEGFA bispecific antibody VP101(hG1DM) are currently being assessed in multiple clinical trials. Hemorrhage, hypertension, proteinuria, and gastrointestinal perforation are adverse events frequently reported in clinical studies of bevacizumab. The inventors analyzed the incidence of these adverse events (≥ grade 3) in the clinical study of the anti-PD-1/anti-VEGFA bispecific antibody VP101(hG1DM), and 648 patients from 7 clinical trials (NCT04047290, NCT04597541, NCT05116007, NCT04736823, NCT04900363, NCT04870177, and NCT0518471) were included in the analysis data set. The experimental results are shown in Tables 3 and 4.

As shown in Table 3, the proportions of patients with grade 3 or greater hemorrhage, hypertension, proteinuria, and gastrointestinal perforation in the anti-PD-1/anti-VEGFA bispecific antibody VP101(hG1DM) group were 0.5%, 4.6%, 1.1%, and 0.3%, respectively, which were lower than those in the bevacizumab group (the data of bevacizumab were from the medicament package insert).

In addition, as shown in Table 4, the analysis of immune-related adverse events (irAEs) in these trials showed that the number of safety events of the anti-PD-1/anti-VEGFA bispecific antibody VP101(hG1DM) was numerically lower relative to the published safety data of nivolumab (the data of nivolumab were from the medicament package insert).

These data showed that the anti-PD-1/anti-VEGFA bispecific antibody VP101(hG1DM) has good *in vivo* safety.

**Table 3: Incidence of hemorrhage, hypertension, proteinuria, and gastrointestinal perforation in clinical trials**

| Adverse events (≥ grade 3) | Bevacizum ab (N = 4463) (%) | VP101(hG1D M) (N=156) (%) | VP101(hG1D M) in combination with chemotherapeut ic drugs (N = 316) (%) | Total VP101(hG1D M) (N = 648) (%) |
|---|---|---|---|---|
| Hemorrhage | 0.4-7 | 0.6 | 0.3 | 0.5 |
| Hypertension | 5-18 | 7.1 | 3.2 | 4.6 |
| Proteinuria | 0.7-7 | 3.2 | 0.3 | 1.1 |
| Gastrointestinal perforation | 0.3-3 | 0.6 | 0 | 0.3 |

**Table 4: Incidence of irAEs in clinical trials**

| irAE | Nivolumab (N=1994) | VP101(hG1D M) (N=156) (%) | VP101(hG1D M) in combination with chemotherape utic drugs (N = 316) (%) | Total VP101(hG1 DM) (N = 648) (%) |
|---|---|---|---|---|
| Immune-mediated pneumonia | 3.1% | 0% | 2.2% | 1.5% |
| Immune-mediated colitis | 2.9% | 1.3% | 0.3% | 0.6% |
| Immune-mediated hepatotoxicity | 1.8% | 3.2% | 1.3% | 1.7% |
| Hyperthyroidism | 2.7% | 5.1% | 4.7% | 4.6% |
| Hypothyroidism | 8% | 12.8% | 9.8% | 10.2% |
| Immune-mediated cutaneous toxicity | 9% | 7.1% | 7.6% | 7.7% |

Although the specific embodiments of the present disclosure have been described in detail, those skilled in the art will understand that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. Use of a bispecific antibody in preparing a medicament for treating or preventing brain metastases from non-small cell lung cancer, wherein
the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

2. The use according to claim 1, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

3. The use according to any one of claims 1 to 2, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

4. The use according to claim 1, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

5. The use according to any one of claims 1 and 4, wherein the bispecific antibody is selected from any one of the following (7)-(12):
(7)
an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

6. The use according to any one of claims 1 to 5, wherein
the immunoglobulin is of a human IgG1 subtype,
wherein, according to the EU numbering system, a heavy chain constant region of the immunoglobulin comprises one of the following mutation combinations:
L234A and L235A; or
L234A, L235A, and G237A.

7. The use according to any one of claims 1 to 3 and 6, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

8. The use according to any one of claims 1 to 7, wherein
the first protein functional region and the second protein functional region are linked directly or via a linker; and/or the heavy chain variable region of the single-chain variable fragment and the light chain variable region of the single-chain variable fragment are linked directly or via a linker.

9. The use according to claim 8, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

10. The use according to any one of claims 1 to 9, wherein
the first protein functional region and the second protein functional region are independently 1, 2, or more in number.

11. The use according to any one of claims 1 to 10, wherein
the single-chain variable fragment is two in number, and the two single-chain variable fragments are linked to the C-termini of the two heavy chains of the immunoglobulin, respectively.

12. The use according to any one of claims 1 to 11, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

13. The use according to any one of claims 1 to 12, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

14. The use according to any one of claims 1 to 13, wherein the bispecific antibody is selected from any one of the following (13)-(16):
(13)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9 or SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18;
(14)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18;
(15)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19;
(16)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

15. The use according to any one of claims 1 to 14, wherein the use is use of a combination of the bispecific antibody with at least one chemotherapeutic drug in preparing a medicament for treating or preventing brain metastases from non-small cell lung cancer.

16. The use according to claim 15, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

17. The use according to any one of claims 1 to 16, wherein the use is **characterized by** one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

18. The use according to any one of claims 1 to 17, wherein the combination is:
the bispecific antibody, pemetrexed, and carboplatin; or
the bispecific antibody, paclitaxel, and carboplatin.

19. The use according to claim 18, wherein the use is **characterized by** one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

20. The use according to any one of claims 1 to 19, wherein the brain metastases from non-small cell lung cancer are selected from one or more of brain metastases from squamous non-small cell lung cancer, brain metastases from non-squamous non-small cell lung cancer, and brain metastases from lung adenocarcinoma;
preferably, a patient with brain metastases from non-small cell lung cancer has PD-L1 TPS ≥ 50%, 1% < PD-L1 TPS ≥ 49%, or PD-L1 TPS ≤ 1%.

21. A bispecific antibody, wherein the bispecific antibody is for use in treating or preventing brain metastases from non-small cell lung cancer, wherein
the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively;
and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

22. The bispecific antibody according to claim 21, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

23. The bispecific antibody according to any one of claims 21 to 22, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

24. The bispecific antibody according to claim 21, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

25. The bispecific antibody according to any one of claims 21 and 24, wherein the bispecific antibody is selected from any one of the following (7)-(12):
(7)
an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

26. The bispecific antibody according to any one of claims 21 to 25, wherein the immunoglobulin is of a human IgG1 subtype,
wherein, according to the EU numbering system, a heavy chain constant region of the immunoglobulin comprises one of the following mutation combinations:
L234A and L235A; or
L234A, L235A, and G237A.

27. The bispecific antibody according to any one of claims 21 to 23 and 26, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

28. The bispecific antibody according to any one of claims 21 to 27, wherein the first protein functional region and the second protein functional region are linked directly or via a linker; and/or the heavy chain variable region of the single-chain variable fragment and the light chain variable region of the single-chain variable fragment are linked directly or via a linker.

29. The bispecific antibody according to claim 28, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

30. The bispecific antibody according to any one of claims 21 to 29, wherein the first protein functional region and the second protein functional region are independently 1, 2, or more in number.

31. The bispecific antibody according to any one of claims 21 to 30, wherein the single-chain variable fragment is two in number, and the two single-chain variable fragments are linked to the C-termini of the two heavy chains of the immunoglobulin, respectively.

32. The bispecific antibody according to any one of claims 21 to 31, wherein the single-chain variable fragment is independently a sequential linkage of:
heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

33. The bispecific antibody according to any one of claims 21 to 32, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

34. The bispecific antibody according to any one of claims 21 to 33, wherein the bispecific antibody is selected from any one of the following (13)-(16):
(13)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9 or SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19; preferably, amino acid sequences of the first linker and the second linker are each set forth in SEQ ID NO: 18;
(14)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18;
(15)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19;
(16)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

35. The bispecific antibody according to any one of claims 21 to 34, wherein the bispecific antibody is administered in combination with at least one chemotherapeutic drug.

36. The bispecific antibody according to claim 35, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

37. The bispecific antibody according to any one of claims 21 to 36, wherein the bispecific antibody is **characterized by** one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

38. The bispecific antibody according to any one of claims 21 to 37, wherein the bispecific antibody is administered in combination with pemetrexed and carboplatin; or
the bispecific antibody is administered in combination with paclitaxel and carboplatin.

39. The bispecific antibody according to claim 38, wherein the bispecific antibody is **characterized by** one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

40. The bispecific antibody according to any one of claims 21 to 39, wherein the brain metastases from non-small cell lung cancer are selected from one or more of brain metastases from squamous non-small cell lung cancer, brain metastases from non-squamous non-small cell lung cancer, and brain metastases from lung adenocarcinoma;
preferably, a patient with brain metastases from non-small cell lung cancer has PD-L1 TPS ≥ 50%, 1% < PD-L1 TPS ≥ 49%, or PD-L1 TPS ≤ 1%.

41. A method for treating or preventing brain metastases from non-small cell lung cancer, comprising administering to a subject in need an effective amount of a bispecific antibody, wherein
the bispecific antibody comprises:
a first protein functional region targeting VEGFA, and
a second protein functional region targeting PD-1,
wherein
the first protein functional region is an immunoglobulin, and the second protein functional region is a single-chain variable fragment, wherein the immunoglobulin comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively;
and, the single-chain variable fragment comprises a heavy chain variable region and a light chain variable region, wherein the heavy chain variable region comprises HCDR1-HCDR3 having the amino acid sequences set forth in SEQ ID NOs: 34-36, respectively, and the light chain variable region comprises LCDR1-LCDR3 having the amino acid sequences set forth in SEQ ID NOs: 37-39, respectively;
or,
the first protein functional region is a single-chain variable fragment, and the second protein functional region is an immunoglobulin, wherein the single-chain variable fragment comprises a heavy chain variable region and a light chain variable region, wherein the heavy chain variable region comprises HCDR1-HCDR3 having the amino acid sequences set forth in SEQ ID NOs: 28-30, respectively, and the light chain variable region comprises LCDR1-LCDR3 having the amino acid sequences set forth in SEQ ID NOs: 31-33, respectively; and, the immunoglobulin comprises a heavy chain variable region and a light chain variable region, wherein the heavy chain variable region comprises HCDR1-HCDR3 having the amino acid sequences set forth in SEQ ID NOs: 34-36, respectively, and the light chain variable region of the immunoglobulin comprises LCDR1-LCDR3 having the amino acid sequences set forth in SEQ ID NOs: 37-39, respectively.

42. The method according to claim 41, wherein
the first protein functional region is an immunoglobulin, and the second protein functional region is a single-chain variable fragment, wherein an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is selected from SEQ ID NO: 5 and SEQ ID NO: 9, and an amino acid sequence of the light chain variable region of the single-chain variable fragment is selected from SEQ ID NO: 7, SEQ ID NO: 11, and SEQ ID NO: 17.

43. The method according to any one of claims 41 to 42, wherein the bispecific antibody is selected from any one of the following (1)-(6):
(1) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 7;
(2) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 11;
(3) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 5, and an amino acid sequence of the light chain variable region of the single-chain variable fragment is set forth in SEQ ID NO: 17;
(4) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 7;
(5) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 11; and
(6) an amino acid sequence of the heavy chain variable region of the immunoglobulin is set forth in SEQ ID NO: 1, and an amino acid sequence of the light chain variable region of the immunoglobulin is set forth in SEQ ID NO: 3; and, an amino acid sequence of the heavy chain variable region of the single-chain variable fragment is set forth in SEQ ID NO: 9, and an amino acid sequence of the light chain variable region of the single-chain variable fragment is set forth in SEQ ID NO: 17.

44. The method according to claim 41, wherein
the first protein functional region is a single-chain variable fragment, and the second protein functional region is an immunoglobulin, wherein an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and, an amino acid sequence of the heavy chain variable region of the immunoglobulin is selected from SEQ ID NO: 5 and SEQ ID NO: 9, and an amino acid sequence of the light chain variable region of the immunoglobulin is selected from SEQ ID NO: 7, SEQ ID NO: 11, and SEQ ID NO: 17.

45. The method according to any one of claims 41 and 44, wherein the bispecific antibody is selected from any one of the following (7)-(12):
(7)
an amino acid sequence of the heavy chain variable region of the immunoglobulin is set forth in SEQ ID NO: 5, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(8)
an amino acid sequence of the heavy chain variable region of the immunoglobulin is set forth in SEQ ID NO: 5, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(9)
an amino acid sequence of the heavy chain variable region of the immunoglobulin is set forth in SEQ ID NO: 5, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(10)
an amino acid sequence of the heavy chain variable region of the immunoglobulin is set forth in SEQ ID NO: 9, and an amino acid sequence of the light chain variable region of the immunoglobulin is set forth in SEQ ID NO: 7; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3;
(11)
an amino acid sequence of the heavy chain variable region of the immunoglobulin is set forth in SEQ ID NO: 9, and an amino acid sequence of the light chain variable region of the immunoglobulin is set forth in SEQ ID NO: 11; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3; and
(12)
an amino acid sequence of the heavy chain variable region of the immunoglobulin is set forth in SEQ ID NO: 9, and an amino acid sequence of the light chain variable region of the immunoglobulin is set forth in SEQ ID NO: 17; and, an amino acid sequence of the heavy chain variable region of the single-chain variable fragment is set forth in SEQ ID NO: 1, and an amino acid sequence of the light chain variable region of the single-chain variable fragment is set forth in SEQ ID NO: 3.

46. The method according to any one of claims 41 to 45, wherein
the immunoglobulin is of a human IgG1 subtype,
wherein, according to the EU numbering system, a heavy chain constant region of the immunoglobulin comprises one of the following mutation combinations:
L234A and L235A; or
L234A, L235A, and G237A.

47. The method according to any one of claims 41 to 43 and 46, wherein
an amino acid sequence of the heavy chain of the immunoglobulin is set forth in SEQ ID NO: 24, and an amino acid sequence of the light chain of the immunoglobulin is set forth in SEQ ID NO: 26;
an amino acid sequence of the heavy chain of the immunoglobulin is set forth in SEQ ID NO: 20, and an amino acid sequence of the light chain of the immunoglobulin is set forth in SEQ ID NO: 26; or
an amino acid sequence of the heavy chain of the immunoglobulin is set forth in SEQ ID NO: 22, and an amino acid sequence of the light chain of the immunoglobulin is set forth in SEQ ID NO: 26.

48. The method according to any one of claims 41 to 47, wherein
the first protein functional region and the second protein functional region are linked directly or via a linker; and/or the heavy chain variable region of the single-chain variable fragment and the light chain variable region of the single-chain variable fragment are linked directly or via a linker.

49. The method according to claim 48, wherein
the linker is the polypeptide set forth in SEQ ID NO: 43 or a polypeptide formed by concatenating a plurality of (e.g., 2, 3, 4, 5, or 6) polypeptides set forth in SEQ ID NO: 43.

50. The method according to any one of claims 41 to 49, wherein
the first protein functional region and the second protein functional region are independently 1, 2, or more in number.

51. The method according to any one of claims 41 to 50, wherein
the single-chain variable fragment is two in number, and the two single-chain variable fragments are linked to the C-termini of the two heavy chains of the immunoglobulin, respectively.

52. The method according to any one of claims 41 to 51, wherein the single-chain variable fragment is independently a sequential linkage of: heavy chain variable region-linker-light chain variable region (VH-linker-VL), or light chain variable region-linker-heavy chain variable region (VL-linker-VH);
preferably, an amino acid sequence of the single-chain variable fragment is set forth in any one of SEQ ID NOs: 45-48.

53. The method according to any one of claims 41 to 52, wherein the first protein functional region is an anti-VEGFA immunoglobulin, and the second protein functional region is an anti-PD-1 single-chain variable fragment;
preferably, two anti-PD-1 single-chain variable fragment molecules are present, and the two molecules are linked to the C-termini of the two heavy chains of the anti-VEGFA immunoglobulin, respectively;
preferably, the bispecific antibody is a bispecific antibody in the form of IgG-scFv;
preferably, the bispecific antibody is a tetravalent bispecific antibody in the form of IgG-scFv.

54. The method according to any one of claims 41 to 53, wherein the bispecific antibody is selected from any one of the following (13)-(16):
(13)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of a heavy chain variable region of the single-chain variable fragment is set forth in SEQ ID NO: 9 or SEQ ID NO: 5, and an amino acid sequence of a light chain variable region of the single-chain variable fragment is set forth in SEQ ID NO: 17, SEQ ID NO: 7, or SEQ ID NO: 11;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19;
preferably, amino acid sequences of the first linker and the second linker are each set forth in SEQ ID NO: 18;
(14)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of a heavy chain variable region of the single-chain variable fragment is set forth in SEQ ID NO: 9, and an amino acid sequence of a light chain variable region of the single-chain variable fragment is set forth in SEQ ID NO: 17;
the single-chain variable fragment is linked to the C-termini of the two heavy chains of the immunoglobulin, respectively;
the first protein functional region and the second protein functional region are linked via a first linker; the heavy chain variable region of the single-chain variable fragment and the light chain variable region of the single-chain variable fragment are linked via a second linker; the first linker and the second linker are identical or different;
preferably, amino acid sequences of the first linker and the second linker are independently selected from SEQ ID NO: 18 and SEQ ID NO: 19; preferably, amino acid sequences of the first linker and the second linker are each set forth in SEQ ID NO: 18;
(15)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain variable region of the immunoglobulin is set forth in SEQ ID NO: 1, and an amino acid sequence of a light chain variable region of the immunoglobulin is set forth in SEQ ID NO: 3;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19;
(16)
the bispecific antibody comprises:
a first protein functional region targeting VEGFA, and
a second protein functional region targeting PD-1;
the first protein functional region is 1 in number, and the second protein functional region is 2 in number,
wherein the first protein functional region is an immunoglobulin, and the second protein functional region is a single-chain variable fragment;
an amino acid sequence of a heavy chain of the immunoglobulin is set forth in SEQ ID NO: 24, SEQ ID NO: 20, or SEQ ID NO: 22, and an amino acid sequence of a light chain of the immunoglobulin is set forth in SEQ ID NO: 26;
an amino acid sequence of the single-chain variable fragment is set forth in any one of SEQ ID NOs: 45-48;
the single-chain variable fragment is linked to the C-termini of the two heavy chains of the immunoglobulin, respectively;
the first protein functional region and the second protein functional region are linked via a first linker;
preferably, the first linker is selected from SEQ ID NO: 18 and SEQ ID NO: 19.

55. The method according to any one of claims 41 to 54, wherein the bispecific antibody is administered in combination with at least one chemotherapeutic drug.

56. The method according to claim 55, wherein
the chemotherapeutic drug is one or more selected from a tyrosine kinase inhibitor, a platinum-based drug (e.g., cisplatin or carboplatin), pemetrexed, paclitaxel, docetaxel, temozolomide, and fotemustine.

57. The method according to any one of claims 41 to 56, wherein the method is **characterized by** one or more of the following (1) to (4):
(1) a unit dose of the bispecific antibody is 100 mg-4000 mg, 200 mg-3000 mg, 200 mg-2000 mg, 300 mg-2000 mg, 400 mg-2000 mg, 500 mg-2000 mg, or 1000 mg-2000 mg;
(2) a single administration dose of the bispecific antibody is 1-50 mg, 10 mg-50 mg, 10 mg-40 mg, 10 mg-30 mg, 10 mg-20 mg, 10 mg-15 mg, 15 mg-30 mg, or 15 mg-20 mg per kg body weight;
(3) the bispecific antibody is administered once every 1 week, every 2 weeks, every 3 weeks, or every 4 weeks;
(4) the bispecific antibody is administered by intravenous drip infusion, intravenous injection, or intraperitoneal injection.

58. The method according to any one of claims 41 to 57, wherein
the bispecific antibody is administered in combination with pemetrexed and carboplatin; or
the bispecific antibody is administered in combination with paclitaxel and carboplatin.

59. The method according to claim 58, wherein the method is **characterized by** one or more of the following (1) to (3):
(1) a single administration dose of pemetrexed is 10-1500 mg/m², 100-1000 mg/m², 200-900 mg/m², 300-800 mg/m², 400-700 mg/m², or 500-600 mg/m² based on a body surface area (m²) of each subject; preferably, pemetrexed is administered once every 1 week, every 2 weeks, every 3 weeks, or every 4 weeks;
(2) a single administration dose of carboplatin is independently AUC 2-6 min*mg/mL or AUC 2.5-5 min*mg/mL; preferably, carboplatin is administered once every 1 week, every 2 weeks, every 3 weeks, or every 4 weeks;
(3) a single administration dose of paclitaxel is 67.5-175 mg/m², 87.5-175 mg/m², or 131-175 mg/m² based on a body surface area (m²) of each subject; preferably, paclitaxel is administered once every 1 week, every 2 weeks, every 3 weeks, or every 4 weeks.

60. The method according to any one of claims 41 to 59, wherein the brain metastases from non-small cell lung cancer are selected from one or more of brain metastases from squamous non-small cell lung cancer, brain metastases from non-squamous non-small cell lung cancer, and brain metastases from lung adenocarcinoma;
preferably, a patient with brain metastases from non-small cell lung cancer has PD-L1 TPS ≥ 50%, 1% < PD-L1 TPS ≥ 49%, or PD-L1 TPS ≤ 1%.
